# EUROPEAN PATENT SPECIFICATION

(11) **EP 3 873 524 B1**
(45) Date of publication and mention of the grant of the patent: **26.02.2025**
(21) Application number: 19878291.4
(22) Date of filing: 30.10.2019
(51) Int. Cl.: A61K 39/395, A61K 51/00, A61P 35/00

(54) **METHODS OF TREATMENT USING ANTI-CD123 IMMUNOCONJUGATES**
BEHANDLUNGSVERFAHREN UNTER VERWENDUNG VON ANTI-CD123-IMMUNOKONJUGATEN
PROCÉDÉS DE TRAITEMENT FAISANT APPEL À DES IMMUNOCONJUGUÉS ANTI-CD123

(30) Priority: 30.10.2018 US 201862752832 P; 12.06.2019 US 201962860565 P; 31.07.2019 US 201962881137 P
(43) Date of publication of application: 08.09.2021
(73) Proprietor: ImmunoGen, Inc., Waltham, MA 02451 (US)
(72) Inventor: ZWEIDLER-MCKAY, Patrick, Lincoln, Massachusetts 01773 (US); CULM-MERDEK, Kerry, Natick, Massachusetts 01760 (US); SLOSS, Callum, Wakefield, Massachusetts 01880 (US); ROMANELLI, Angela, Manchester-by-the-Sea, Massachusetts 01944 (US)
(74) Representative: Simmons & Simmons LLP (Munich)
(86) International application number: PCT/US2019/058824
(87) International publication number: WO 2020/092533

(56) References cited:
- WO-A2-2018/098258
- US-A1- 2017 152 321
- US-A1- 2017 152 321
- US-A1- 2018 169 261
- TOLCHER A.W.: "Antibody drug conjugates: lessons from 20 years of clinical experience", ANNALS OF ONCOLOGY, vol. 27, no. 12, 1 December 2016 (2016-12-01), pages 2168 - 2172, XP093147201, ISSN: 0923-7534, DOI: 10.1093/annonc/mdw424
- DAVER NAVAL G ET AL: "A Phase I, First-in-Human Study Evaluating the Safety and Preliminary Antileukemia Activity of IMGN632, a Novel CD123-Targeting Antibody-Drug Conjugate, in Patients with Relapsed/Refractory Acute Myeloid Leukemia and Other CD123-Positive Hematologic Malignancies", BLOOD, AMERICAN SOCIETY OF HEMATOLOGY, US, vol. 132, 29 November 2018 (2018-11-29), pages 27, XP086593652, ISSN: 0006-4971, DOI: 10.1182/BLOOD-2018-99-112955
- TOLCHER A.W.: "Antibody drug conjugates: lessons from 20 years of clinical experience", ANNALS OF ONCOLOGY, vol. 27, no. 12, 1 December 2016 (2016-12-01), pages 2168 - 2172, XP093147201, ISSN: 0923-7534, DOI: 10.1093/annonc/mdw424
- DAVER NAVAL G ET AL: "A Phase I, First-in-Human Study Evaluating the Safety and Preliminary Antileukemia Activity of IMGN632, a Novel CD123-Targeting Antibody-Drug Conjugate, in Patients with Relapsed/Refractory Acute Myeloid Leukemia and Other CD123-Positive Hematologic Malignancies", BLOOD, AMERICAN SOCIETY OF HEMATOLOGY, US, vol. 132, 29 November 2018 (2018-11-29), pages 27, XP086593652, ISSN: 0006-4971, DOI: 10.1182/BLOOD-2018-99-112955
- TOLCHER A.W.: "Antibody drug conjugates: lessons from 20 years of clinical experience", ANNALS OF ONCOLOGY, vol. 27, no. 12, 1 December 2016 (2016-12-01), pages 2168 - 2172, XP093147201, ISSN: 0923-7534, DOI: 10.1093/annonc/mdw424
- DAVER NAVAL G ET AL: "A Phase I, First-in-Human Study Evaluating the Safety and Preliminary Antileukemia Activity of IMGN632, a Novel CD123-Targeting Antibody-Drug Conjugate, in Patients with Relapsed/Refractory Acute Myeloid Leukemia and Other CD123-Positive Hematologic Malignancies", BLOOD, AMERICAN SOCIETY OF HEMATOLOGY, US, vol. 132, 29 November 2018 (2018-11-29), pages 27, XP086593652, ISSN: 0006-4971, DOI: 10.1182/BLOOD-2018-99-112955

## Description

### FIELD

The present disclosure generally relates to uses of anti-CD123 immunoconjugates for the treatment of diseases, such as cancer. Provided herein are therapeutically effective dosing regimens that minimize unwanted side-effects.

### BACKGROUND

Cancer is one of the leading causes of death in the developed world, with over one million people diagnosed with cancer and 500,000 deaths per year in the United States alone. Overall it is estimated that more than 1 in 3 people will develop some form of cancer during their lifetime.

CD123 is the alpha-subunit of the interleukin-3 receptor (IL-3Rα). CD123 expression is low on normal hematopoietic stem cells (Testa et al., Biomark Res., 10;2(1):4.(2014), Jordan et al., Leukemia, 14(10):1777-84 (2000)). However, CD123 is overexpressed in multiple hematological malignancies of both myeloid and lymphoid origins, including acute myeloid leukemia (AML), myelodysplastic syndrome (MDS), B-cell acute lymphoblastic leukemia (B-ALL), chronic myeloid leukemia in blast crisis/phase (BP-CML), and blastic plasmacytoid dendritic cell neoplasm (BPDCN) (Testa 2014). Interleukin-3 is produced by activated T-lymphocytes. IL-3 together with other growth factors stimulates the development and mediates the survival of a wide range of hematopoietic cells in bone marrow (Testa 2014). CD123 levels on normal hematopoietic stem cells are very low, but early common myeloid progenitors express higher CD123 levels (Testa 2014, Jordan 2000). Medium to high expression of CD123 on normal tissues is limited to rare populations of white blood cells, such as plasmacytoid dendritic cells and basophils (Jordan 2000, Testa 2014).

Acute myeloid leukemia is the most common form of acute leukemia among adults and accounts for the largest number of deaths from leukemias in the United States. In 2017, an estimated 21,380 people will be diagnosed with AML per year and 10,590 patients will die of the disease (Siegel et al., CA Cancer J Clin. 2017;67(1):7-30 (2017)). The median age of diagnosis is 66 years. Frontline chemotherapy in AML is reported to induce complete response (CR) in 70%-80% of patients who are 60 years of age or younger and in approximately 50% of older patients. "Fit" patients are judged to be able to tolerate intensive treatment, are often younger (< 60 years), and typically receive one to two cycles of induction with "7 + 3," a combination of cytarabine and anthracycline, typically daunorubicin. Following this, these fit patients may receive high-dose cytarabine for one or more cycles and may receive a stem cell transplant. Standard induction and post-induction therapies result in a median duration of remission of approximately one year and potential cures in 25%-35% of the patients. "Unfit" patients, often older, typically receive azacitidine, a hypomethylating agent. The majority of AML patients will eventually relapse, and AML salvage regimens offer poor outcomes with significant toxicity. Thus, novel therapies with limited toxicity in this relapsed population are needed.

Blastic plasmacytoid dendritic cell neoplasm is a rare, aggressive hematologic malignancy derived from myeloid dendritic cell precursors, which often manifests with skin lesions in addition to lymph node, blood, and bone marrow involvement. Characterized by CD4, CD56, and CD123 expression among other markers, BPDCN blasts express high levels of CD123. Unfortunately, there is no standard of care for BPDCN, with both acute lymphoblastic leukemia (ALL) and AML regimens used in frontline treatment. Despite CR rates of 47%-86% in frontline disease, median overall survival is approximately 12-16 months. The majority of BPDCN patients will eventually relapse with no standard treatment options.

Acute lymphoblastic leukemia is a rare, aggressive hematologic malignancy derived from lymphoid precursors, which often manifests with lymph node, blood, and bone marrow involvement. B-cell acute lymphoblastic leukemia and some T-cell acute lymphoblastic leukemia blasts express CD123 at levels similar to AML blasts. Although initial remission rates are high, long-term survival rates are 35%-40% in patients less than 60 years of age, and less than 10% for older patients (Goldstone 2008). Patients with relapsed ALL have several chemotherapeutic options, as well as immunotherapy with United States Food and Drug Administration-approved anti-CD19 bispecific blinatumomab. However, long-term survival remains poor for these patients. US20170152321 provides methods for inhibiting or reducing an IL-3 receptor alpha subunit alpha (IL3Rα)-expressing cell population, the methods comprising contacting a population of IL3Rα-expressing cells (e.g., cancer cells and/or cancer stem cells) with an antibody that binds to IL3Rα. It is disclosed that an antibody can be administered at a dose of 0.001 mg/kg per day to 100 mg/kg per day or an antibody conjugate can be administered at a dose of 0.001 mg/kg per day to 10 mg/kg per day. WO2018/098258 discloses a method for preparing a cell-binding agent-cytotoxic agent conjugate comprising an imine-containing cytotoxic agent bearing a maleimide group covalently linked to a cell-binding agent.

Given the inability of currently available therapeutics to treat many hematological malignancies, there is a need for more effective interventions.

### SUMMARY

Provided herein is an anti-CD123 immunoconjugate comprising an anti-CD123 antibody as defined in the claims or antigen-binding fragment thereof linked to a cytotoxic agent as defined in the claims for use in a method for treating a hematologic malignancy in a human subject, the method comprising administering to the subject the anti-CD123 immunoconjugate comprising an anti-CD123 antibody or antigen-binding fragment thereof linked to a cytotoxic agent, wherein the immunoconjugate is administered at a dose of 0.045 mg/kg. The immunoconjugate is administered to the subject once in a 21-day cycle.

In some embodiments, the immunoconjugate is administered for one cycle.

In some embodiments, the immunoconjugate is administered for more than one cycle. In some embodiments, the immunoconjugate is administered for at least 2 cycles, at least 3 cycles, at least 4 cycles, at least 5 cycles, at least 6 cycles, at least 7 cycles, at least 8 cycles, at least 9 cycles, or at least 10 cycles. In some embodiments, the immunoconjugate is administered for about 2-4 cycles, about 2-6 cycles, about 2-8 cycles, or about 2-10 cycles.

In some embodiments, the hematological malignancy is a relapsed hematological malignancy. In some embodiments, the relapse is a first relapse. In some embodiments, the hematological malignancy is a refractory hematological malignancy. In some embodiments, the hematological malignancy is a primary refractory hematological malignancy. In some embodiments, the hematological malignancy is acute myeloid leukemia (AML), myelodysplastic syndrome (MDS), B-cell acute lymphoblastic leukemia (B-ALL), chronic myeloid leukemia in blast crisis/phase (BP-CML), and blastic plasmacytoid dendritic cell neoplasm (BPDCN). In some embodiments, the hematological malignancy is AML. In some embodiments, the AML is relapsed AML. In some embodiments, the AML is refractory AML. In some embodiments, the hematological malignancy is BPDCN. In some embodiments, the BPDCN is relapsed BPDCN. In some embodiments, the BPDCN is refractory BPDCN. In some embodiments, the BPDCN is front line BPDCN. In some embodiments, the hematological malignancy is ALL. In some embodiments, the ALL is relapsed ALL. In some embodiments, the ALL is refractory ALL. In some embodiments, the hematological malignancy is chronic myelomonocytic leukemia (CMML). In some embodiments, the CMML is relapsed CMML. In some embodiments, the CMML is refractory CMML. In some embodiments, the hematological malignancy is myelofibrosis (MF). In some embodiments, the MF is relapsed MF. In some embodiments, the MF is refractory MF. In some embodiments, the hematological malignancy is MDS. In some embodiments, the MDS is relapsed MDS. In some embodiments, the MDS is refractory MDS.

In some embodiments, the subject is a pediatric subject, e.g., a pediatric subject with BPDCN, ALL, or AML).

In some embodiments, the subject has an Eastern Cooperative Oncology Group (ECOG) performance status of ≤1. In some embodiments, the subject has an adverse European LeukemiaNet (ELN) genetic risk classification, e.g., a ASXL1, RUNX1, and/or FLT3-ITD mutation. In some embodiments, the subject has previously failed SL-401. In some embodiments, the hematological malignancy is refractory to (CLAG-M).

In some embodiments, the hematological malignancy is a CD123-expressing hematological malignancy. In some embodiments, CD123 has been detected in a sample obtained from the hematological malignancy prior to the administration. In some embodiments, the CD123 was detected using flow cytometry.

In some embodiments, the methods disclosed herein further comprise detecting CD123 in a sample obtained from the hematological malignancy prior to the administration. In some embodiments, at least 80% of cells in the hematological malignancy express CD123. In some embodiments, CD123 has been detected in at least 80% of cells in a sample obtained from the hematological malignancy prior to the administration. In some embodiments, the methods disclosed herein further comprise detecting CD123 in at least 80% of cells in a sample obtained from the hematological malignancy prior to the administration. In some embodiments, the subject has an absolute neutrophil count of greater than 500/µL.

In some embodiments, the subject received at least one prior line of therapy. In some embodiments, the subject received at least two prior lines of therapy. In some embodiments, the subject received at least three prior lines of therapy. In some embodiments, the subject has received no more than three prior lines of therapy. In some embodiments, the subject has received four prior lines of therapy. In some embodiments, the subject has received five prior lines of therapy. In some embodiments, the subject has received no more than five prior lines of therapy. In some embodiments, the subject has previously received a stem cell transplant.

In some embodiments, the administration decreases bone marrow blasts in the subject.

In some embodiments, the subject has been pretreated with a corticosteroid prior to administration of the immunoconjugate. In some embodiments, the methods disclosed herein further comprise pre-treating the subject with a corticosteroid prior to administration of the immunoconjugate. In some embodiments, the subject has been pretreated with diphenhydramine, acetaminophen, paracetamol, dexamethasone, or a combination thereof.

In some embodiments, the immunoconjugate is administered intravenously.

In some embodiments, the method further comprises administering a reduced dose of the immunoconjugate after a dose-limiting toxicity has occurred in the subject and has been reduced to baseline or ≤ Grade 2.

The anti-CD123 antibody or antigen-binding fragment in the immunoconjugate comprises: (a) a heavy chain variable region CDR1 comprising the amino acid sequence of SEQ ID NO: 5; a heavy chain variable region CDR2 comprising the amino acid sequence of SEQ ID NO: 6; and a heavy chain variable region CDR3 comprising the amino acid sequence of SEQ ID NO: 7; and (b) a light chain variable region CDR1 comprising the amino acid sequence of SEQ ID NO: 8; a light chain variable region CDR2 comprising the amino acid sequence of SEQ ID NO: 9; and a light chain variable region CDR3 comprising the amino acid sequence selected from the group consisting of: SEQ ID NO: 10.

In some embodiments, the anti-CD123 antibody or antigen-binding fragment in the immunoconjugate comprises a VH comprising the amino acid sequence set forth in SEQ ID NO:1 and a VL comprising the amino acid sequence set forth in SEQ ID NO: 2. In some embodiments, the anti-CD123 antibody or antigen-binding fragment in the immunoconjugate comprises a heavy chain constant region and/or a light chain constant region. In some embodiments, the heavy chain constant region comprises a human immunoglobulin IgG₁ heavy chain constant region and/or wherein the light chain constant region comprises a human immunoglobulin IgGx light chain constant region. In some embodiments, the anti-CD123 antibody or antigen-binding fragment in the immunoconjugate comprises a heavy chain comprising the amino acid sequence set forth in SEQ ID NO:3 and a light chain comprising the amino acid sequence set forth in SEQ ID NO:4. In some embodiments, the anti-CD123 antibody or antigen-binding fragment in the immunoconjugate is a full length antibody. In some embodiments, the anti-CD123 antibody or antigen-binding fragment in the immunoconjugate is an antigen binding fragment. The cytotoxic agent in the immunoconjugate is a DNA alkylating agent, which is an indolino-benzodiazepine (IGN) DNA-alkylator. In some embodiments, the IGN DNA-alkylator is DGN549-C. In some embodiments, the immunoconjugate comprises a peptide linker. In some embodiments, the immunoconjugate is administered in a pharmaceutical composition comprising immunoconjugates with the following structure: 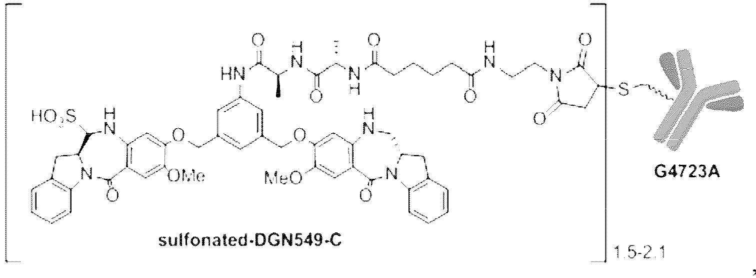 wherein G4723A comprises a heavy chain comprising the amino acid sequence set forth in SEQ ID NO:3 and a light chain comprising the amino acid sequence set forth in SEQ ID NO:4.

### BRIEF DESCRIPTION OF THE FIGURES

**FIG. 1** shows the study design schema of IMGN632 (an anti-CD123 immunoconjugate) in a clinical trial dose escalation phase and in maximum tolerated dose (MTD) expansion cohorts. R/R: relapsed or refractory; AML: acute myeloid leukemia; BPDCN: blastic plasmacytoid dendritic cell neoplasm; CRC: clinical research center; RP2D: recommended Phase 2 dose; ALL: acute lymphoblastic leukemia.
**FIG. 2** shows the best percent change in bone marrow blasts in patients treated with IMGN632 who achieved progressive disease (PD; checkered boxes), stable disease (SD; open boxes) complete remission (CR), complete remission with incomplete recovery CR/CRi; angled line boxes), partial remission (PR; inverse angled line box), and minimal residual disease (MRD; gray boxes). Each bar represents best percent change in bone marrow blasts in an individual patient, and the number above or below the bar is the cohort number (see Tables 5 and 6) that the patient was in.
**FIG. 3** shows the percentage of CD123-positive leukemic cells in patients treated with IMGN632.
**FIG. 4** shows the percentage of CD123 receptor saturation in patients in Cohorts 1-6.
**FIG. 5** shows the concentration of IMGN632 in patients in Cohorts 1-6.
**FIG. 6A** shows the chemical structure for IMGN632. IMGN632 is composition comprising immunoconjugates containing the anti-CD123 G4723A antibody linked to the cytotoxic payload DGN549-C in sodium bisulfite. The majority of the immunoconjugate in the composition is in the sulfonated version shown in **FIG. 6A****.**
**FIG. 6B** shows an unsulfonated form of the immunoconjugate containing the anti-CD123 G4723A antibody linked to the cytotoxic payload DGN549-C (the mono-imine structure), which can also be present in an IMGN632 composition.

### DETAILED DESCRIPTION

### Definitions

The terms "cancer" and "cancerous" refer to or describe the physiological condition in mammals in which a population of cells are characterized by unregulated cell growth. Examples of cancer include, but are not limited to, carcinoma, lymphoma, blastoma, sarcoma, and leukemia. "Tumor" and "neoplasm" refer to one or more cells that result from excessive cell growth or proliferation, either benign (noncancerous) or malignant (cancerous) including pre-cancerous lesions. A cancer as disclosed herein can be a hematological malignancy. Examples of hematological malignancies include, for example, acute myeloid leukemia (AML), chronic myeloid leukemia (CML), myelodysplastic syndrome (MDS), acute lymphoblastic leukemia (ALL) such as B-cell acute lymphoblastic leukemia (B-ALL), T-cell acute lymphoblastic leukemia (T ALL), mixed-lineage leukemia ALL (MLL-ALL), B-cell precursor ALL (BCP-ALL), Ph+ ALL, Ph-like ALL, chronic lymphocytic leukemia (CLL), chronic myeloid leukemia in blast crisis/phase (BP-CML), and blastic plasmacytoid dendritic cell neoplasm (BPDCN). Additional examples of "cancer" include, B-cell lymphomas including NHL, precursor B-cell lymphoblastic leukemia/lymphoma and mature B-cell neoplasms, such as B-cell chronic lymphocytic leukemia (CLL)/small lymphocytic lymphoma (SLL), B-cell prolymphocytic leukemia, lymphoplasmacytic lymphoma, mantle cell lymphoma (MCL), follicular lymphoma (FL), including low- grade, intermediate-grade and high-grade FL, cutaneous follicle center lymphoma, marginal zone B-cell lymphoma (MALT type, nodal and splenic type), hairy cell leukemia, diffuse large B-cell lymphoma, Burkitt's lymphoma, plasmacytoma, plasma cell myeloma, post-transplant lymphoproliferative disorder, Waldenstrom's macroglobulinemia, and anaplastic large-cell lymphoma (ALCL).The cancer can be a cancer that expresses CD123 ("CD123-expressing cancer").

The terms "cancer cell," "tumor cell," and grammatical equivalents refer to the total population of cells derived from a tumor or a pre-cancerous lesion, including both non-tumorigenic cells, which comprise the bulk of the tumor cell population, and tumorigenic stem cells (cancer stem cells). As used herein, the term "tumor cell" will be modified by the term "non-tumorigenic" when referring solely to those tumor cells lacking the capacity to renew and differentiate to distinguish those tumor cells from cancer stem cells.

A "refractory" cancer is one that progresses even though an anti-tumor treatment, such as a chemotherapy, is administered to the cancer patient. An example of a refractory cancer is one which is platinum refractory.

A "relapsed" cancer is one in which the cancer or the signs and symptoms of a cancer returns after a period of improvement.

A "complete response" or "complete remission" or "CR" indicates the disappearance of all signs of tumor or cancer in response to treatment. This does not always mean the cancer has been cured. A "CRi" refers to a morphologically complete remissions with an incomplete hematological (blood count) recovery. A "CRMRD-" refers to a complete recovery without measurable residual disease.

A "CRc" or "complete remission clinical" indicates no evidence of disease with some skin changes not indicative of active disease. A "CR with partial hematologic recovery" or "CRh" refers to a hematologic recovery which is defined as a patient having no signs of leukemia, but one or more blood counts (e.g., platelets and neutrophils) have not returned to normal levels (e.g., absolute neutrophil count (ANC) of over 500/µl and platelet count over 50,000/µl).

A "partial response" or "PR" refers to a decrease in the size or volume of one or more tumors or lesions, or in the extent of cancer in the body, in response to treatment.

"Progressive disease" refers to the appearance of one more new lesions or tumors and/or the unequivocal progression of existing non-target lesions. Progressive disease can also refer to a tumor growth of more than 20% since treatment began, either due to an increases in mass or in spread of the tumor.

The term "antibody" means an immunoglobulin molecule that recognizes and specifically binds to a target, such as a protein, polypeptide, peptide, carbohydrate, polynucleotide, lipid, or combinations of the foregoing through at least one antigen recognition site within the variable region of the immunoglobulin molecule. As used herein, the term "antibody" encompasses intact polyclonal antibodies, intact monoclonal antibodies, chimeric antibodies, humanized antibodies, human antibodies, fusion proteins comprising an antibody, and any other modified immunoglobulin molecule so long as the antibodies exhibit the desired biological activity. An antibody can be of any the five major classes of immunoglobulins: IgA, IgD, IgE, IgG, and IgM, or subclasses (isotypes) thereof (e.g. IgG1, IgG2, IgG3, IgG4, IgA1 and IgA2), based on the identity of their heavy-chain constant domains referred to as alpha, delta, epsilon, gamma, and mu, respectively. The different classes of immunoglobulins have different and well known subunit structures and three-dimensional configurations. Antibodies can be naked or conjugated to other molecules such as toxins, radioisotopes, etc.

The term "antibody fragment" refers to a portion of an intact antibody with a sufficient positive charge to bind to a cation exchange resin. An "antigen-binding fragment" refers to a portion of an intact antibody that binds to an antigen and has a sufficient positive charge to bind to a cation exchange resin. An antigen-binding fragment can contain the antigenic determining variable regions of an intact antibody. Examples of antibody fragments include, but are not limited to Fab, Fab', F(ab')2, and Fv fragments, linear antibodies, and single chain antibodies.

The term "cysteine engineered" antibody or antigen-binding fragment thereof includes an antibody or antigen-binding fragment thereof with at least one cysteine ("Cys") that is not normally present at a given residue of the antibody or antigen-binding fragment thereof light chain or heavy chain. Such Cys, which may also be referred to as "engineered Cys," can be engineered using any conventional molecular biology or recombinant technology (e.g., by replacing the coding sequence for a non-Cys residue at the target residue with a coding sequence for Cys). For example, if the original residue is Ser with a coding sequence of 5'-UCU-3', the coding sequence can be mutated (e.g., by site-directed mutagenesis) to 5'-UGU-3', which encodes Cys. In certain embodiments, the Cys engineered antibody or antigen-binding fragment thereof has an engineered Cys in the heavy chain. In certain embodiments, the engineered Cys is in or near the CH3 domain of the heavy chain. In certain embodiments, the engineered Cys is at residue 442 of the heavy chain (EU/OU numbering; EU index, Kabat et al, Sequences of Proteins of Immunological Interest, 5th Ed., NIH publication No. 91-3242, 1991). In certain embodiments, the Fc region comprises a cysteine at one or more of positions 239, 282, 289, 297, 312, 324, 330, 335, 337, 339, 356, 359, 361, 383, 384, 398, 400, 440, 422, and 442, as numbered by the EU index. In certain embodiments, any one or more of the following residues may be substituted with cysteine: V205 (Kabat numbering) of the light chain; A118 (EU numbering) of the heavy chain; and S400 (EU numbering) of the heavy chain Fc region. In certain embodiments, the variable light chain domain, e.g., of an scFv, has a cysteine at Kabat position 100. In certain embodiments, the variable heavy chain domain, e.g. of an scFv, has a cysteine at Kabat position 44. Cysteine engineered antibodies may be generated as described, e.g., in U.S. Pat. No. 7,521,541, U.S. Pat. No. 7,855,275, U.S. Published Application No. 20110033378 and WO 2011/005481.

A "monoclonal" antibody or antigen-binding fragment thereof refers to a homogeneous antibody or antigen-binding fragment population involved in the highly specific recognition and binding of a single antigenic determinant, or epitope. This is in contrast to polyclonal antibodies that typically include different antibodies directed against different antigenic determinants. The term "monoclonal" antibody or antigen-binding fragment thereof encompasses both intact and full-length monoclonal antibodies as well as antibody fragments (such as Fab, Fab', F(ab')2, Fv), single chain (scFv) mutants, fusion proteins comprising an antibody portion, and any other modified immunoglobulin molecule comprising an antigen recognition site. Furthermore, "monoclonal" antibody or antigen-binding fragment thereof refers to such antibodies and antigen-binding fragments thereof made in any number of manners including but not limited to by hybridoma, phage selection, recombinant expression, and transgenic animals.

The term "humanized" antibody or antigen-binding fragment thereof refers to forms of non-human (e.g. murine) antibodies or antigen-binding fragments that are specific immunoglobulin chains, chimeric immunoglobulins, or fragments thereof that contain minimal non-human (e.g., murine) sequences. Typically, humanized antibodies or antigen-binding fragments thereof are human immunoglobulins in which residues from the complementary determining region (CDR) are replaced by residues from the CDR of a non-human species (e.g. mouse, rat, rabbit, hamster) that have the desired specificity, affinity, and capability ("CDR grafted") (Jones et al., Nature 321:522-525 (1986); Riechmann et al., Nature 332:323-327 (1988); Verhoeyen et al., Science 239:1534-1536 (1988)). In some instances, the Fv framework region (FR) residues of a human immunoglobulin are replaced with the corresponding residues in an antibody or fragment from a non-human species that has the desired specificity, affinity, and capability. The humanized antibody or antigen-binding fragment thereof can be further modified by the substitution of additional residues either in the Fv framework region and/or within the replaced non-human residues to refine and optimize antibody or antigen-binding fragment thereof specificity, affinity, and/or capability. In general, the humanized antibody or antigen-binding fragment thereof will comprise substantially all of at least one, and typically two or three, variable domains containing all or substantially all of the CDR regions that correspond to the non-human immunoglobulin whereas all or substantially all of the FR regions are those of a human immunoglobulin consensus sequence. The humanized antibody or antigen-binding fragment thereof can also comprise at least a portion of an immunoglobulin constant region or domain (Fc), typically that of a human immunoglobulin. Examples of methods used to generate humanized antibodies are described in U.S. Pat. 5,225,539; Roguska et al., Proc. Natl. Acad. Sci., USA, 91(3):969-973 (1994), and Roguska et al., Protein Eng. 9(10):895-904 (1996). In some embodiments, a "humanized antibody" is a resurfaced antibody.

A "variable region" of an antibody refers to the variable region of the antibody light chain or the variable region of the antibody heavy chain, either alone or in combination. The variable regions of the heavy and light chain each consist of four framework regions (FR) connected by three complementarity determining regions (CDRs) also known as hypervariable regions. The CDRs in each chain are held together in close proximity by the FRs and, with the CDRs from the other chain, contribute to the formation of the antigen-binding site of antibodies. There are at least two techniques for determining CDRs: (1) an approach based on cross-species sequence variability (i.e., Kabat et al., Sequences of Proteins of Immunological Interest, (5th ed., 1991, National Institutes of Health, Bethesda Md.), "Kabat"); and (2) an approach based on crystallographic studies of antigen-antibody complexes (Al-lazikani et al, J. Molec. Biol. 273:927-948 (1997)). In addition, combinations of these two approaches are sometimes used in the art to determine CDRs.

The Kabat numbering system is generally used when referring to a residue in the variable domain (approximately residues 1-107 of the light chain and residues 1-113 of the heavy chain) (e.g., Kabat et al., Sequences of Immunological Interest. (5th Ed., 1991, National Institutes of Health, Bethesda, Md.) ("Kabat").

The amino acid position numbering as in Kabat, refers to the numbering system used for heavy chain variable domains or light chain variable domains of the compilation of antibodies in Kabat et al. (Sequences of Immunological Interest. (5th Ed., 1991, National Institutes of Health, Bethesda, Md.), "Kabat"). Using this numbering system, the actual linear amino acid sequence can contain fewer or additional amino acids corresponding to a shortening of, or insertion into, a FR or CDR of the variable domain. For example, a heavy chain variable domain can include a single amino acid insert (residue 52a according to Kabat) after residue 52 of H2 and inserted residues (e.g. residues 82a, 82b, and 82c, etc. according to Kabat) after heavy chain FR residue 82. The Kabat numbering of residues can be determined for a given antibody by alignment at regions of homology of the sequence of the antibody with a "standard" Kabat numbered sequence. Chothia refers instead to the location of the structural loops (Chothia and Lesk, J. Mol. Biol. 196:901-917 (1987)). The end of the Chothia CDR-H1 loop when numbered using the Kabat numbering convention varies between H32 and H34 depending on the length of the loop (this is because the Kabat numbering scheme places the insertions at H35A and H35B; if neither 35A nor 35B is present, the loop ends at 32; if only 35A is present, the loop ends at 33; if both 35A and 35B are present, the loop ends at 34). The AbM hypervariable regions represent a compromise between the Kabat CDRs and Chothia structural loops, and are used by Oxford Molecular's AbM antibody modeling software.

| Loop | Kabat | AbM | Chothia |
|---|---|---|---|
| L1 | L24-L34 | L24-L34 | L24-L34 |
| L2 | L50-L56 | L50-L56 | L50-L56 |
| L3 | L89-L97 | L89-L97 | L89-L97 |
| H1 | H31-H35B | H26-H35B | H26-H32..34 |
| | | (Kabat Numbering) | |
| H1 | H31-H35 | H26-H35 | H26-H32 |
| | | (Chothia Numbering) | |
| H2 | H50-H65 | H50-H58 | H52-H56 |
| H3 | H95-H102 | H95-H102 | H95-H102 |

The term "human" antibody or antigen-binding fragment thereof means an antibody or antigen-binding fragment thereof produced by a human or an antibody or antigen-binding fragment thereof having an amino acid sequence corresponding to an antibody or antigen-binding fragment thereof produced by a human made using any technique known in the art. This definition of a human antibody or antigen-binding fragment thereof includes intact or full-length antibodies and fragments thereof.

The term "chimeric" antibodies or antigen-binding fragments thereof refers to antibodies or antigen-binding fragments thereof wherein the amino acid sequence is derived from two or more species. Typically, the variable region of both light and heavy chains corresponds to the variable region of antibodies or antigen-binding fragments thereof derived from one species of mammals (e.g. mouse, rat, rabbit, etc.) with the desired specificity, affinity, and capability while the constant regions are homologous to the sequences in antibodies or antigen-binding fragments thereof derived from another (usually human) to avoid eliciting an immune response in that species.

The term "epitope" or "antigenic determinant" are used interchangeably herein and refer to that portion of an antigen capable of being recognized and specifically bound by a particular antibody. When the antigen is a polypeptide, epitopes can be formed both from contiguous amino acids and noncontiguous amino acids juxtaposed by tertiary folding of a protein. Epitopes formed from contiguous amino acids are typically retained upon protein denaturing, whereas epitopes formed by tertiary folding are typically lost upon protein denaturing. An epitope typically includes at least 3, and more usually, at least 5 or 8-10 amino acids in a unique spatial conformation.

"Binding affinity" generally refers to the strength of the sum total of noncovalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (Kd). Affinity can be measured by common methods known in the art, including those described herein. Low-affinity antibodies generally bind antigen slowly and tend to dissociate readily, whereas high-affinity antibodies generally bind antigen faster and tend to remain bound longer. A variety of methods of measuring binding affinity are known in the art, any of which can be used for purposes of the present disclosure. Specific illustrative embodiments are described in the following.

"Or better" when used herein to refer to binding affinity refers to a stronger binding between a molecule and its binding partner. "Or better" when used herein refers to a stronger binding, represented by a smaller numerical Kd value. For example, an antibody which has an affinity for an antigen of "0.6 nM or better", the antibody's affinity for the antigen is <0.6 nM, i.e. 0.59 nM, 0.58 nM, 0.57 nM etc. or any value less than 0.6 nM.

By "specifically binds," it is generally meant that an antibody binds to an epitope via its antigen binding domain, and that the binding entails some complementarity between the antigen binding domain and the epitope. According to this definition, an antibody is said to "specifically bind" to an epitope when it binds to that epitope, via its antigen binding domain more readily than it would bind to a random, unrelated epitope. The term "specificity" is used herein to qualify the relative affinity by which a certain antibody binds to a certain epitope. For example, antibody "A" may be deemed to have a higher specificity for a given epitope than antibody "B," or antibody "A" may be said to bind to epitope "C" with a higher specificity than it has for related epitope "D."

By "preferentially binds," it is meant that the antibody specifically binds to an epitope more readily than it would bind to a related, similar, homologous, or analogous epitope. Thus, an antibody which "preferentially binds" to a given epitope would more likely bind to that epitope than to a related epitope, even though such an antibody may cross-react with the related epitope.

The terms "polypeptide," "peptide," and "protein" are used interchangeably herein to refer to polymers of amino acids of any length. The polymer can be linear or branched, it can comprise modified amino acids, and it can be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, etc.), as well as other modifications known in the art. It is understood that, because the polypeptides of this disclosure are based upon antibodies, in certain embodiments, the polypeptides can occur as single chains or associated chains.

The term "immunoconjugate" or "conjugate" as used herein refers to a compound or a derivative thereof that is linked to a cell binding agent (i.e., an anti-CD123 antibody or fragment thereof) and is defined by a generic formula: C-A, wherein C = cytotoxin (e.g., such as an indolino-benzodiazepine (IGN) DNA-alkylator (e.g., DGN549-C)) and A = antibody or antigen-binding fragment thereof, e.g., an anti-CD123 antibody or antibody fragment. An immunoconjugate can optionally contain a linker and be defined by the generic formula C-L-A, wherein C = cytotoxin, L = linker, and A = antibody or antigen-binding fragment thereof, e.g., an anti-CD123 antibody or antibody fragment. Immunoconjugates can also be defined by the generic formula in reverse order: C-A or A-L-C. Immunoconjugates can also contain multiple cytotoxins (C) per antibody or antigen-binding fragment thereof (A) or multiple cytotoxins (C) and linkers (L) per antibody or antigen-binding fragment thereof (A).

A "linker" is any chemical moiety that is capable of linking a compound, usually a drug (such as IGN DNA-alkylators), to a cell-binding agent (such as an anti-CD123 antibody or a fragment thereof) in a stable, covalent manner. Linkers can be susceptible to or be substantially resistant to acid-induced cleavage, light-induced cleavage, peptidase-induced cleavage, esterase-induced cleavage, and disulfide bond cleavage, at conditions under which the compound or the antibody remains active. Suitable linkers are well known in the art and include, for example, disulfide groups, thioether groups, acid labile groups, photolabile groups, peptidase labile groups and esterase labile groups. Linkers also include charged linkers, and hydrophilic forms thereof as described herein and known in the art. In some embodiments disclosed herein, the linker is a peptide linker.

The phrase "pharmaceutically acceptable" indicates that the substance or composition must be compatible chemically and/or toxicologically, with the other ingredients comprising a formulation, and/or the mammal being treated therewith.

The term "pharmaceutical formulation" refers to a preparation which is in such form as to permit the biological activity of the active ingredient to be effective, and which contains no additional components which are unacceptably toxic to a subject to which the formulation would be administered. The formulation can be sterile.

An "effective amount" of an antibody, immunoconjugate, or other drug as disclosed herein is an amount sufficient to carry out a specifically stated purpose. An "effective amount" can be determined empirically and in a routine manner, in relation to the stated purpose.

The term "fit AML" as used herein refers to a subject having AML who is eligible for intensive therapy. The measures for determining a subject with fit AML include, e.g., physical performance (as determined by e.g., the Eastern Cooperative Oncology Group performance status (ECOG PS), the Karnofsky performance status (KPS), and the short physical performance battery (SPPB)), comorbid conditions (as determined by the Charlson comorbidity index (CCI) or the hematopoietic cell transplantation-specific comorbidity index (HCT-CI)), cognitive function, and prognostic models (including but not limited to, cytogenetic group, age, white blood cell count, LDH, type of AML). In some cases, a fit AML subject is a subject at the age of 60 or under the age of 60.

The term "unfit AML" as used herein refers to a subject having AML who is ineligible for intensive therapy. The measures for determining a subject with unfit AML include, e.g., physical performance (as determined by e.g., the Eastern Cooperative Oncology Group performance status (ECOG PS), the Karnofsky performance status (KPS), and the short physical performance battery (SPPB)), comorbid conditions (as determined by the Charlson comorbidity index (CCI) or the hematopoietic cell transplantation-specific comorbidity index (HCT-CI)), cognitive function, and prognostic models (including but not limited to, cytogenetic group, age, white blood cell count, LDH, type of AML). In some cases, an unfit AML subject is a subject over the age of 60.

The term "therapeutically effective amount" refers to an amount of an antibody, immunoconjugate, or other drug effective to "treat" a disease or disorder in a subject or mammal. In the case of cancer, the therapeutically effective amount of the drug can reduce the number of cancer cells; reduce the tumor size or burden; inhibit (i.e., slow to some extent and in a certain embodiment, stop) cancer cell infiltration into peripheral organs; relieve to some extent one or more of the symptoms associated with the cancer; and/or result in a favorable response such as complete remission (CR), complete remission with incomplete recovery (CRi); CR without minimal residual disease (CRMRD-); complete remission clinical (CRc); morphologic leukemia-free state; partial remission (PR); and decrease in progressive disease (PD). See the definition herein of "treating". To the extent the drug can prevent growth and/or kill existing cancer cells, it can be cytostatic and/or cytotoxic. A "prophylactically effective amount" refers to an amount effective, at dosages and for periods of time necessary, to achieve the desired prophylactic result. Typically but not necessarily, since a prophylactic dose is used in subjects prior to or at an earlier stage of disease, the prophylactically effective amount will be less than the therapeutically effective amount.

The term "respond favorably" generally refers to causing a beneficial state in a subject. With respect to cancer treatment, the term refers to providing a therapeutic effect on the subject. Positive therapeutic effects in cancer can be measured in a number of ways (See, W.A. Weber, J. Nucl. Med. 50:1S-10S (2009)). A favorable response can be assessed, for example, by complete remission (CR), complete remission with incomplete recovery (CRi); CR without minimal residual disease (CRMRD-); complete remission clinical (CRc); morphologic leukemia-free state; partial remission (PR); a decrease in progressive disease (PD), or any combination thereof.

The terms "IL-3Rα," "Interleukine-3 Receptor alpha," and "CD123," as used interchangeably herein, refer to mammalian CD123 polypeptides, including, but not limited to, native CD123 polypeptides and isoforms of CD123 polypeptides, unless otherwise indicated. The terms encompass "full-length," unprocessed CD123 polypeptides as well as any form of CD123 polypeptide that results from processing within the cell. The term also encompasses naturally occurring variants of CD123, e.g., those encoded by splice variants and allelic variants. The CD123 polypeptides described herein can be isolated from a variety of sources, such as from human tissue types or from another source, or prepared by recombinant or synthetic methods. Where specifically indicated, "CD123" can be used to refer to a nucleic acid that encodes a CD123 polypeptide. Human CD123 sequences are known and include, for example, those sequences associated with NCBI reference numbers NP_002174 & NM_002183 (protein and nucleic acid sequences for human CD123 variant 1), and NP_001254642 & NM_001267713 (protein and nucleic acid sequences for human CD123 variant 2). As used herein, the term "human CD123" refers to CD123 comprising the sequence of SEQ ID NO:11 or SEQ ID NO:12.

1 MVLLWLTLLL IALPCLLQTK EDPNPPITNL RMKAKAQQLT WDLNRNVTDI ECVKDADYSM

61 PAVNNSYCQF GAISLCEVTN YTVRVANPPF STWILFPENS GKPWAGAENL TCWIHDVDFL

121 SCSWAVGPGA PADVQYDLYL NVANRRQQYE CLHYKTDAQG TRIGCRFDDI SRLSSGSQSS

181 HILVRGRSAA FGIPCTDKFV VFSQIEILTP PNMTAKCNKT HSFMHWKMRS HFNRKFRYEL

241 QIQKRMQPVI TEQVRDRTSF QLLNPGTYTV QIRARERVYE FLSAWSTPQR FECDQEEGAN

301 TRAWRTSLLI ALGTLLALVC VFVICRRYLV MQRLFPRIPH MKDPIGDSFQ NDKLVVWEAG

361 KAGLEECLVT EVQVVQKT (SEQ ID NO:11)

1 MVLLWLTLLL IALPCLLQTK EGGKPWAGAE NLTCWIHDVD FLSCSWAVGP GAPADVQYDL

61 YLNVANRRQQ YECLHYKTDA QGTRIGCRFD DISRLSSGSQ SSHILVRGRS AAFGIPCTDK

121 FVVFSQIEIL TPPNMTAKCN KTHSFMHWKM RSHFNRKFRY ELQIQKRMQP VITEQVRDRT
181 SFQLLNPGTY TVQIRARERV YEFLSAWSTP QRFECDQEEG ANTRAWRTSL LIALGTLLAL
241 VCVFVICRRY LVMQRLFPRI PHMKDPIGDS FQNDKLVVWE AGKAGLEECL VTEVQVVQKT
(SEQ ID NO:12)

The term "anti-CD123 antibody" or "an antibody that binds to CD123" refers to an antibody that is capable of binding CD123 with sufficient affinity such that the antibody is useful as a diagnostic and/or therapeutic agent in targeting CD123 (e.g., the antibody in IMGN632). The extent of binding of an anti-CD123 antibody to an unrelated, non-CD123 protein can be less than about 10% of the binding of the antibody to CD123 measured, e.g., by a radioimmunoassay (RIA).

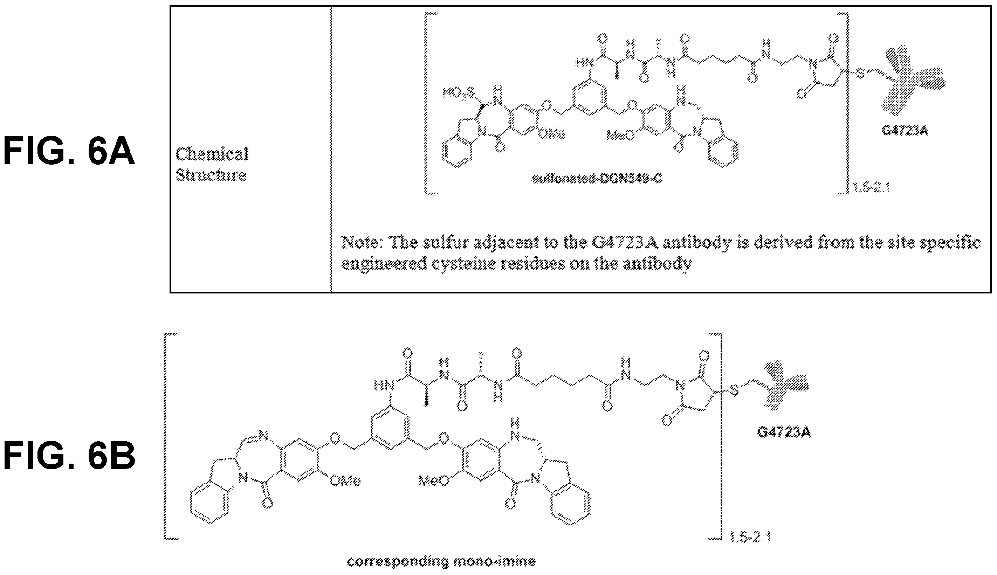

The term "IMGN632" refers to the immunoconjugate composition shown in FIGs. 6A and 6B. The immunoconjugate composition comprises immunoconjugates comprising an average of 1.5 to 2.1 DGN549-C cytotoxic agents per huCD123-6Gv4.7 ("G4723A") antibody in a sulfonated version (Figure 6A). The immunoconjugate composition can also comprise the unsulfonated immunoconjugate (the mono-imine structure shown in Figure 6B).

As used in the present disclosure and claims, the singular forms "a," "an," and "the" include plural forms unless the context clearly dictates otherwise.

It is understood that wherever embodiments are described herein with the language "comprising," otherwise analogous embodiments described in terms of "consisting of" and/or "consisting essentially of" are also provided.

The term "and/or" as used in a phrase such as "A and/or B" herein is intended to include both "A and B," "A or B," "A," and "B." Likewise, the term "and/or" as used in a phrase such as "A, B, and/or C" is intended to encompass each of the following embodiments: A, B, and C; A, B, or C; A or C; A or B; B or C; A and C; A and B; B and C; A (alone); B (alone); and C (alone).

### Anti-CD123 Immunoconjugates

The methods described herein provide methods of administering immunoconjugates that specifically bind to CD123. These agents are referred to herein as "CD123-immunoconjugates" or "anti-CD123-immunoconjugates." Such immunoconjugates comprise an anti-CD123 antibody or antigen-binding fragment thereof and a drug (e.g., a cytotoxic agent). The drug (e.g., a cytotoxic agent) can be attached to the anti-CD123 antibody or antigen-binding fragment thereof by a linker.

In some embodiments, the anti-CD123 antibodies or antigen-binding fragments thereof are humanized antibodies or antigen-binding fragments thereof. In some embodiments, the humanized antibody or fragment is a resurfaced antibody or antigen-binding fragment thereof. In other embodiments, the antibodies or antigen-binding fragments thereof is a fully human antibody or antigen-binding fragment thereof.

Provided herein is an immunoconjugate represented by the following formula: 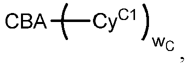 wherein CBA is an anti-CD123 antibody or antigen-binding fragment or polypeptide, covalently linked to Cy^{C1} through a cysteine residue; and W_{C} is 1 or 2.

In the above formula, Cy^{C1} is represented by the following formulae: 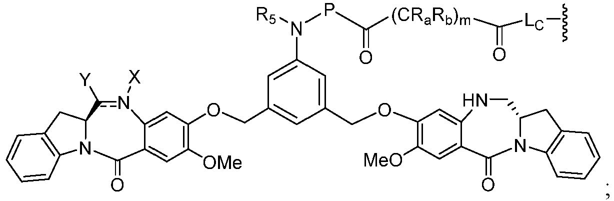 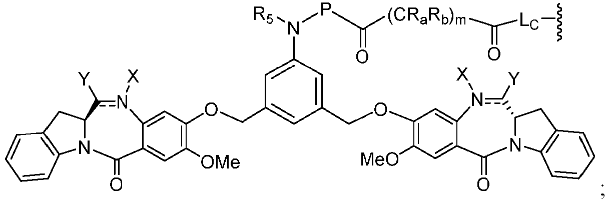 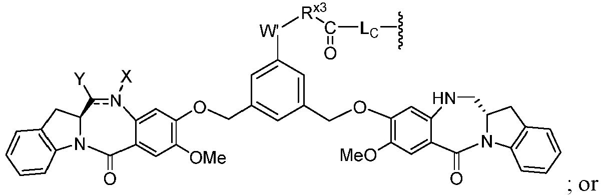 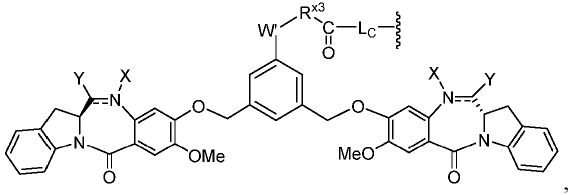 or a pharmaceutically acceptable salt thereof, wherein the double line -̅ -̅ between N and C represents a single bond or a double bond, provided that when it is a double bond, X is absent and Y is -H or a (C₁-C₄)alkyl; and when it is a single bond, X is -H or an amine protecting moiety, Y is -OH or -SO₃M, and M is H⁺ or a cation;
R₅ is -H or a (C₁-C₃)alkyl;
P is an amino acid residue or a peptide containing 2 to 20 amino acid residues;
Rₐ and R_{b}, for each occurrence, are independently -H, (C₁-C₃)alkyl, or a charged substituent or an ionizable group Q;
W' is -NR^{e'},
R^{e'} is -(CH₂-CH₂-O)ₙ-R^{k};
n is an integer from 2 to 6;
R^{k} is -H or -Me;
R^{x3} is a (C₁-C₆)alkyl; and,
L_{C} is represented by 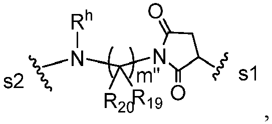 s1 is the site covalently linked to CBA, and s2 is the site covalently linked to the -C(=O)- group on Cy^{C1}; wherein:
   R₁₉ and R₂₀, for each occurrence, are independently -H or a (C₁-C₃)alkyl;
   m" is an integer between 1 and 10; and
   R^{h} is -H or a (C₁-C₃)alkyl.

In certain embodiments, Rₐ and R_{b} are both H; and R₅ is H or Me.

In certain embodiments, P is a peptide containing 2 to 5 amino acid residues. For example, P may be selected from Gly-Gly-Gly, Ala-Val, Val-Ala, Val-Cit, Val-Lys, Phe-Lys, Lys-Lys, Ala-Lys, Phe-Cit, Leu-Cit, Ile-Cit, Trp, Cit, Phe-Ala, Phe-N⁹-tosyl-Arg, Phe-N⁹-nitro-Arg, Phe-Phe-Lys, D-Phe-Phe-Lys, Gly-Phe-Lys, Leu-Ala-Leu, Ile-Ala-Leu, Val-Ala-Val, Ala-Leu-Ala-Leu, β-Ala-Leu-Ala-Leu, Gly-Phe-Leu-Gly, Val-Arg, Arg-Val, Arg-Arg, Val-D-Cit, Val-D-Lys, Val-D-Arg, D-Val-Cit, D-Val-Lys, D-Val-Arg, D-Val-D-Cit, D-Val-D-Lys, D-Val-D-Arg, D-Arg-D-Arg, Ala-Ala, Ala-D-Ala, D-Ala-Ala, D-Ala-D-Ala, Ala-Met, and Met-Ala. In certain embodiments, P is Gly-Gly-Gly, Ala-Val, Ala-Ala, Ala-D-Ala, D-Ala-Ala, or D-Ala-D-Ala. In certain embodiments, Q is -SO₃M.

In certain embodiments, R₁₉ and R₂₀ are both H; and m" is an integer from 1 to 6.

In certain embodiments, -L_{C}- is represented by the following formula:

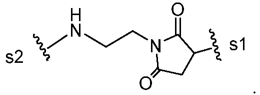

In certain embodiments, the immunoconjugate is represented by the following formulae: 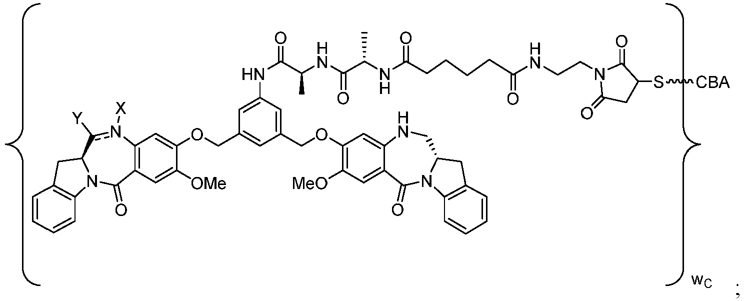 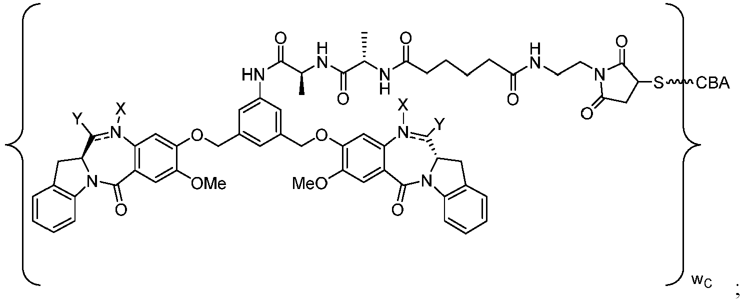 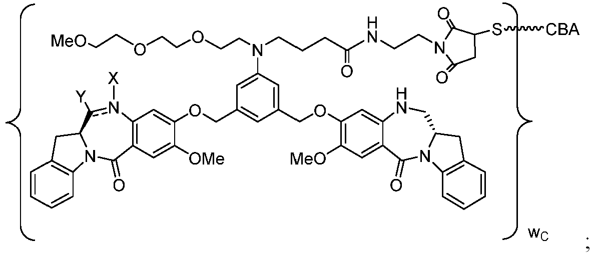 or 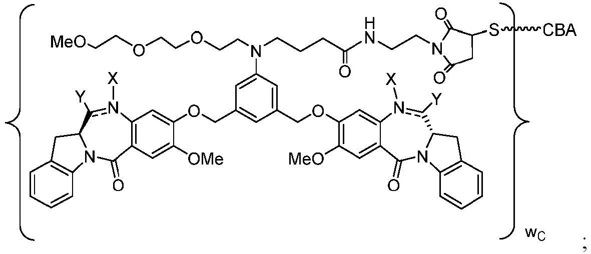 or a pharmaceutically acceptable salt thereof, wherein the double line -̅ -̅ between N and C represents a single bond or a double bond, provided that when it is a double bond, X is absent and Y is -H, and when it is a single bond, X is -H, and Y is -OH or -SO₃M.

In the present invention, an anti-CD123 antibody or antigen-binding fragment thereof is in an immunoconjugate for use in the present methods. Anti-CD123 antibodies or antigen-binding fragments thereof have been described (see e.g., US Patent No. 10,077,313 B2). The anti-CD123 antibody or antigen-binding fragment thereof can be the huCD123-6Gv4.7 ("G4723A") antibody (see WO 2017/004025, WO 2017/004026, and PCT/US2018/052212 ) or can contain sequences of the G4723A antibody, e.g., as shown below in Tables 1-3. An anti-CD123 antibody or antigen-binding fragment thereof for use in the methods provided herein comprises variable heavy chain CDR-1, CDR-2, and CDR-3 comprising the sequences of SEQ ID NOs: 5, 6, and 7, respectively and variable light chain CDR-1, CDR-2, and CDR-3 comprising the sequences of SEQ ID NOs: 8, 9, and 10, respectively. An anti-CD123 antibody or antigen-binding fragment thereof for use in the methods provided herein can comprise a variable heavy chain domain comprising the sequence set forth in SEQ ID NO: 1. An anti-CD123 antibody or antigen-binding fragment thereof for use in the methods provided herein can comprise a variable light chain domain comprising the sequence set forth in SEQ ID NO:2. An anti-CD123 antibody or antigen-binding fragment thereof for use in the methods provided herein can comprise a variable heavy chain domain comprising the sequence set forth in SEQ ID NO:1 and a variable light chain domain comprising the sequence set forth in SEQ ID NO:2. An anti-CD123 antibody or antigen-binding fragment thereof for use in the methods provided herein can comprise a heavy chain comprising the sequence set forth in SEQ ID NO:3. An anti-CD123 antibody or antigen-binding fragment thereof for use in the methods provided herein can comprise a light chain comprising the sequence set forth in SEQ ID NO:4. An anti-CD123 antibody or antigen-binding fragment thereof for use in the methods provided herein can comprise a heavy chain comprising the sequence set forth in SEQ ID NO:3 and a light chain comprising the sequence set forth in SEQ ID NO:4.

**Table 1. huCD123-6Gv4.7 Heavy and Light Chain Variable Regions**

| **Name** | **Sequence** |
|---|---|
| huCD123-6Gv7 Heavy Chain | QVQLVQSGAEVKKPGASVKVSCKASGYIFTSSIMH |
| Variable Region | WVRQAPGQGLEWIGYIKPYNDGTKYNEKFKGRA TLTSDRSTSTAYMELSSLRSEDTAVYYCAREGGND YYDTMDYWGQGTLVTVSS (SEQ ID NO:1) |
| huCD123-6Gv4 Light Chain Variable Region | DIQMTQSPSSLSASVGDRVTITCRASQDINSYLSWF QQKPGKAPKTLIYRVNRLVDGVPSRFSGSGSGNDY TLTISSLQPEDFATYYCLQYDAFPYTFGQGTKVEIK R (SEQ ID NO:2) |

**Table 2. huCD123-6Gv4.7-C442 Full Length Heavy and Light Chain**

| **Name** | **Sequence** |
|---|---|
| huCD123-6Gv7-C442 Full Length Heavy Chain | QVQLVQSGAEVKKPGASVKVSCKASGYIFTSSIMH WVRQAPGQGLEWIGYIKPYNDGTKYNEKFKGRA TLTSDRSTSTAYMELSSLRSEDTAVYYCAREGGND YYDTMDYWGQGTLVTVSSASTKGPSVFPLAPSSKS TSGGTAALGCLVKDYFPEPVTVSWNSGALTSGVHT FPAVLQSSGLYSLSSVVTVPSSSLGTQTYICNVNHKP SNTKVDKKVEPKSCDKTHTCPPCPAPELLGGPSVFL FPPKPKDTLMISRTPEVTCVVVDVSHEDPEVKFNW YVDGVEVHNAKTKPREEQYNSTYRVVSVLTVLHQ DWLNGKEYKCKVSNKALPAPIEKTISKAKGQPREP QVYTLPPSRDELTKNQVSLTCLVKGFYPSDIAVEWE SNGQPENNYKTTPPVLDSDGSFFLYSKLTVDKSRW QQGNVFSCSVMHEALHNHYTQKSLCLSPG (SEQ ID NO:3) |
| huCD123-6Gv4 Full Length Light Chain | DIQMTQSPSSLSASVGDRVTITCRASQDINSYLSWF QQKPGKAPKTLIYRVNRLVDGVPSRFSGSGSGNDY TLTISSLQPEDFATYYCLQYDAFPYTFGQGTKVEIK RTVAAPSVFIFPPSDEQLKSGTASVVCLLNNFYPRE AKVQWKVDNALQSGNSQESVTEQDSKDSTYSLSST LTLSKADYEKHKVYACEVTHQGLSSPVTKSFNRGE C (SEQ ID NO:4) |

**Table 3. huCD123-6Gv4.7 Variable Heavy and Light Chain Complementary Determining Regions**

| **Name** | **Sequence** |
|---|---|
| huCD123-6Gv7 Variable Heavy Chain CDR1 | SSIMH (SEQ ID NO:5) |
| huCD123-6Gv7 Variable Heavy Chain CDR2 | YIKPYNDGTKYNEKFKG (SEQ ID NO:6) |
| huCD123-6Gv7 Variable Heavy Chain CDR3 | EGGNDYYDTMDY (SEQ ID NO:7) |
| huCD123-6Gv4 Variable Light Chain CDR1 | RASQDINSYLS (SEQ ID NO:8) |
| huCD123-6Gv4 Variable Light Chain CDR2 | RVNRLVD (SEQ ID NO:9) |
| huCD123-6Gv4 Variable Light Chain CDR3 | LQYDAFPYT (SEQ ID NO:10) |

An anti-CD123 antibody or antigen-binding fragment thereof for use in the methods provided herein can bind to an epitope within amino acids 205 to 346 of human CD123.

An anti-CD123 antibody or antigen-binding fragment thereof for use in methods provided herein can be recombinantly produced. For example, an anti-CD123 antibody or antigen-binding fragment thereof for use in the methods provided herein can be produced in a mammalian cell line, e.g., a CHO cell.

An anti-CD123 antibody or antigen-binding fragment thereof for use in the methods provided herein can be a cysteine-engineered antibody or fragment. Cysteine-engineered antibodies can be covalently conjugated to cytotoxic agents of interest to generate immunoconjugates.

As used herein, the expression "linked to a cell-binding agent" or "linked to an anti-CD123 antibody or fragment" refers to a conjugate molecule comprising at least one cytotoxic agent bound to a cell-binding agent, e.g., anti-CD123 antibody or fragment, via a suitable linking group, or a precursor thereof. Linkers include, for example, peptide linkers.

An immunoconjugate can contain multiple cytotoxic agents bound to an antibody or antigen-binding fragment thereof. As provided herein, in certain instances, about 1 to about 3 drug molecules e.g., cytotoxic agents, are linked to an anti-CD123 antibody or antigen-binding fragment thereof. In one aspect, an immunoconjugate comprises 1, 2, or 3, cytotoxic agents per antibody or antigen-binding fragment thereof.

A composition comprising immunoconjugates can contain immunoconjugates with varying numbers of cytotoxic agents bound per antibody or antigen-binding fragment thereof. Thus, compositions comprising immunoconjugates can contain an average number of cytotoxic agents bound per antibody or antigen-binding fragment thereof. In one aspect, a pharmaceutical composition comprising anti-CD123 immunoconjugates comprises about 1 to about 3 cytotoxic agents per anti-CD123 antibody or antigen-binding fragment thereof, about 1.5 to about 2.5 cytotoxic agents per anti-CD123 antibody or antigen-binding fragment thereof, about 1.5 to about 2.1 cytotoxic agents per anti-CD123 antibody or antigen-binding fragment thereof, or about 1.5 to about 2.0 cytotoxic agents cytotoxic agents per anti-CD123 antibody or antigen-binding fragment thereof.

In certain instances, a pharmaceutical composition for use in the methods provided herein comprises anti-CD123 immunoconjugates comprising about 1 to about 3 cytotoxic agents per antibody or antigen-binding fragment thereof, for example, wherein the average number of cytotoxic agents per antibody or antigen-binding fragment thereof is from about 1 to about 3 (e.g., 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0).

In certain instances, a pharmaceutical composition for use in the methods provided herein comprises anti-CD123 immunoconjugates with an average of about 1 ± 0.2, about 1.1 ± 0.2, about 1.2 ± 0.2, about 1.3 ± 0.2, about 1.4 ± 0.2, about 1.5 ± 0.2, about 1.6 ± 0.2, about 1.7 ± 0.2, about 1.8 ± 0.2, about 1.9 ± 0.2, about 2.0 ± 0.2, about 2.1 ± 0.2, 2.2 ± 0.2, 2.3 ± 0.2, 2.4 ± 0.2, 2.5 ± 0.2, or 2.6 ± 0.2 drug molecules (e.g., cytotoxic agents) attached per antibody or antigen-binding fragment thereof. In certain aspects, a pharmaceutical composition provided herein comprises anti-CD123 immunoconjugates with an average of about 1.5 to 2.1 drug molecules (e.g., cytotoxic agents) per antibody.

The antibodies or antigen-binding fragments thereof for use in the present disclosure may be linked to cytotoxic agents, for example, through linkage with the Lys side chain amino group, the Cys side chain thiol group, or an oxidized N-terminal Ser/Thr. Cytotoxic agents include, for example, DNA alkylating agents such as indolino-benzodiazepene (IGN) DNA alkylators. In certain instances, an anti-CD123 immunoconjugate for use in the present disclosure comprises DGN549-C.

### Uses and Methods

Anti-CD123-immunoconjugates are useful, for example, in treating hematological malignancies. Accordingly, the present disclosure relates to a dosage regimen for administering an anti-CD123 immunoconjugate (e.g. IMGN632) to a human patient to treat a hematological malignancy. The treatment can result in a decrease in bone marrow blasts.

In the present invention, the anti-CD123 immunoconjugate (e.g., IMGN632) is administered once in a three-week (21-day) cycle.

In certain embodiments, one cycle of treatment is therapeutically effective. In certain embodiments, two cycles of treatment are therapeutically effective. In certain embodiments, one to four cycles of treatment are therapeutically effective.

In some embodiments, patients can be treated for one three-week (21-day) cycle, e.g., wherein the immunoconjugate is administered once in the three-week. In some embodiments, patients can be treated for at least two three-week (21-day) cycles, e.g., wherein the immunoconjugate is administered once per three-week cycle. In some embodiments, patients can be treated for at least three three-week (21-day) cycles e.g., wherein the immunoconjugate is administered once per three-week cycle. In some embodiments, patients can be treated for at least four three-week (21-day) cycles e.g., wherein the immunoconjugate is administered once per three-week cycle. In some embodiments, patients can be treated for at least five three-week (21-day) cycles e.g., wherein the immunoconjugate is administered once per three-week cycle. In some embodiments, patients can be treated for at least six three-week (21-day) cycles e.g., wherein the immunoconjugate is administered once per three-week cycle. In some embodiments, patients can be treated for at least seven three-week (21-day) cycles e.g., wherein the immunoconjugate is administered once per three-week cycle. In some embodiments, patients can be treated for at least eight three-week (21-day) cycles e.g., wherein the immunoconjugate is administered once per three-week cycle. In some embodiments, patients can be treated for at least nine three-week (21-day) cycles e.g., wherein the immunoconjugate is administered once per three-week cycle. In some embodiments, patients can be treated for at least ten three-week (21-day) cycles e.g., wherein the immunoconjugate is administered once per three-week cycle.

In some embodiments, patients can be treated for one to ten three-week (21-day) cycles e.g., wherein the immunoconjugate is administered once per three-week cycle. In some embodiments, patients can be treated for two to ten three-week (21-day) cycles e.g., wherein the immunoconjugate is administered once per three-week cycle. In some embodiments, patients can be treated for three to ten three-week (21-day) cycles e.g., wherein the immunoconjugate is administered once per three-week cycle. In some embodiments, patients can be treated for four to ten three-week (21-day) cycles e.g., wherein the immunoconjugate is administered once per three-week cycle. In some embodiments, patients can be treated for five to ten three-week (21-day) cycles e.g., wherein the immunoconjugate is administered once per three-week cycle.

In the present invention, 0.045 mg/kg of an anti-CD123 immunoconjugate (e.g., IMGN632) is administered once in a three-week (21-day) cycle.

The dosing regimens provided herein can be used to treat a hematological malignancy in a human subject, for example, in a method comprising administering a therapeutically effective amount of a CD123-binding agent to a subject (e.g., a subject in need of treatment). In some embodiments, the hematological malignancy is of myeloid origin. In some embodiments, the hematological malignancy is of lymphoid origin. In some embodiments, the hematological malignancy is of both myeloid and lymphoid origins. In certain embodiments, the hematological malignancy is a B-cell malignancy. In certain embodiments, the hematological malignancy is a CD123-expressing hematological malignancy. In certain embodiments, the hematological malignancy is selected from the group consisting of acute myeloid leukemia (AML), myelodysplastic syndrome (MDS), acute lymphoblastic leukemia (ALL), B-cell acute lymphoblastic leukemia (B-ALL), T-cell acute lymphoblastic leukemia (T ALL), chronic myeloid leukemia in blast crisis/phase (BP-CML), and blastic plasmacytoid dendritic cell neoplasm (BPDCN).

In certain embodiments, the hematological malignancy is a relapsed hematological malignancy. In certain embodiments, the relapse is a first relapse. In certain embodiments, the hematological malignancy is a refractory hematological malignancy. In certain embodiments, the hematological malignancy is a primary refractory hematological malignancy.

In certain embodiments, the hematological malignancy is AML. In certain embodiments, the AML is relapsed AML. In certain embodiments, the AML is refractory AML. In certain embodiments, the AML is not secondary AML. In certain embodiments, the subject with the AML is a pediatric subject.

In certain embodiments, the hematological malignancy is BPDCN. In certain embodiments, the BPDCN is relapsed BPDCN. In certain embodiments, the BPDCN is refractory BPDCN. In certain embodiments, the BPDCN is front line BPDCN. Front line (1L) BPDCN patients are defined as (i) unfit for intensive chemotherapy and/or (ii) not eligible for other approved CD123-targeted therapies, e.g., SL-401. In certain embodiments, the subject with the BPDCN is a pediatric subject.

In certain embodiments, the hematological malignancy is ALL. In certain embodiments, the ALL is relapsed ALL. In certain embodiments, the ALL is refractory ALL. In certain embodiments, the subject with the ALL is a pediatric subject.

In certain embodiments, the hematological malignancy is MDS. In certain embodiments, the MDS is high risk MDS.

In certain embodiments, the hematological malignancy is chronic myelomonocytic leukemia (CMML).

In certain embodiments, the hematological malignancy is myelofibrosis (MF).

In some embodiments, the subject is a pediatric subject. A pediatric subject is less than 18 years old. In some embodiments, a pediatric subject is at least 2 years old and less than 18 years old.

In some embodiments, the subject has an Eastern Cooperative Oncology Group (ECOG) performance status of ≤1.

In some embodiments, the subject has an adverse European LeukemiaNet (ELN) genetic risk classification, e.g., a ASXL1, RUNX1, and/or FLT3-ITD mutation. In some embodiments, the subject has previously failed SL-401. In some embodiments, the hematological malignancy is refractory to (CLAG-M).

In certain embodiments, the hematological malignancy is chemotherapy resistant.

In certain embodiments, the hematological malignancy is chemotherapy sensitive.

In some embodiments, at least about 80% of cells of the hematological malignancy are CD123+.

In some embodiments, it has been determined prior to the administration that at least 80% of cells of the hematological malignancy are CD123+.

In certain instances, the human subject has received at least one prior treatment regimen for the cancer. In certain instances, the human subject has received one prior treatment regimen for the cancer. In certain instances, the human subject has received two prior treatment regimens for the cancer. In certain instances, the human subject has received two prior treatment regimens for the cancer. In certain instances, the human subject has received no more than six prior treatment regimens for the cancer. In certain instances, the human subject has received at least one prior treatment, but no more than six prior treatment regimens for the cancer. In certain instances, the human subject has received no more than three prior treatment regimens for the cancer. In certain instances, the human subject has received at least one prior treatment, but no more than three prior treatment regimens for the cancer. In some embodiments, the subject has previously received a stem cell transplant

As provided herein, anti-CD123 immunoconjugates can be administered in a pharmaceutical composition. In certain instances, a pharmaceutical composition comprises anti-CD123 immunoconjugates (e.g., IMGN632) and a pharmaceutically acceptable vehicle. Accordingly, provided herein are methods of administering pharmaceutical compositions comprising anti-CD123 immunoconjugates (e.g., IMGN632) thereof having the desired degree of purity in a physiologically acceptable carrier, excipient, or stabilizer (Remington's Pharmaceutical Sciences (1990) Mack Publishing Co., Easton, PA). Acceptable carriers, excipients, or stabilizers are nontoxic to recipients at the dosages and concentrations employed. (See, e.g., Gennaro, Remington: The Science and Practice of Pharmacy with Facts and Comparisons: Drugfacts Plus, 20th ed. (2003); Ansel et al., Pharmaceutical Dosage Forms and Drug Delivery Systems, 7th ed., Lippencott Williams and Wilkins (2004); Kibbe et al., Handbook of Pharmaceutical Excipients, 3rd ed., Pharmaceutical Press (2000)). The compositions to be used for *in vivo* administration can be sterile. This is readily accomplished by filtration through, e.g., sterile filtration membranes.

In some embodiments, patients receiving an anti-CD123 immunoconjugate as disclosed herein have received pretreatment with a corticosteroid. Accordingly, in some embodiments, the methods provided herein comprise administering a corticosteroid to a patient prior to administering an anti-CD123 immunoconjugate to the patient. In certain instances, the corticosteroid can be selected from the group consisting of prednisone, prednisolone, methylprednisolone, beclamethasone, betamethasone, dexamethasone, fludrocortisone, hydrocortisone, and triamcinolone. In certain instances the corticosteroid is administered intravenously. In certain instances, the steroid is administered orally.

For example, in some embodiments, patients receiving an anti-CD123 immunoconjugate as disclosed herein have received pretreatment with diphenhydramine. In some embodiments, patients receiving an anti-CD123 immunoconjugate as disclosed herein have received pretreatment with 25-50 mg diphenhydramine. In some embodiments, diphenhydramine is given intravenously. In some embodiments, diphenhydramine is given orally. Accordingly, in some embodiments, the methods provided herein comprise administering diphenhydramine to a patient prior to administering an anti-CD123 immunoconjugate to the patient.

In some embodiments, patients receiving an anti-CD123 immunoconjugate as disclosed herein have received pretreatment with acetaminophen. In some embodiments, patients receiving an anti-CD123 immunoconjugate as disclosed herein have received pretreatment with 325-650 mg acetaminophen. In some embodiments, acetaminophen is given intravenously. In some embodiments, acetaminophen is given orally. Accordingly, in some embodiments, the methods provided herein comprise administering acetaminophen to a patient prior to administering an anti-CD123 immunoconjugate to the patient.

In some embodiments, patients receiving an anti-CD123 immunoconjugate as disclosed herein have received pretreatment with paracetamol. In some embodiments, patients receiving an anti-CD123 immunoconjugate as disclosed herein have received pretreatment with 325-650 mg paracetamol. In some embodiments, paracetamol is given intravenously. In some embodiments, paracetamol is given orally. Accordingly, in some embodiments, the methods provided herein comprise administering paracetamol to a patient prior to administering an anti-CD123 immunoconjugate to the patient.

In some embodiments, patients receiving an anti-CD123 immunoconjugate as disclosed herein have received pretreatment with dexamethasone. In some embodiments, patients receiving anti-CD123 immunoconjugate as disclosed herein have received pretreatment with 8 mg dexamethasone. In some embodiments, dexamethasone is given intravenously. In some embodiments, dexamethasone is given orally. Accordingly, in some embodiments, the methods provided herein comprise administering dexamethasone to a patient prior to administering an anti-CD123 immunoconjugate to the patient.

### EXAMPLES

Examples or parts of examples concerned with subject-matter not encompassed by the claims are for reference.

### Example 1: Phase 1 Study Design

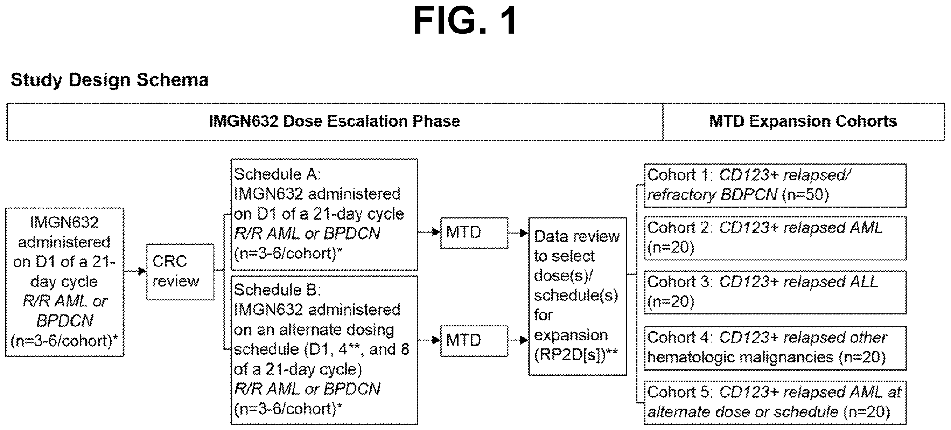

A phase 1, multi-center, open label study of IMGN632 was designed to evaluate the effects of intravenous administration of IMGN632 in adult patients with recurrent or relapsed CD123+ AML and other CD123+ hematologic malignancies. The Phase 1 study schema is provided in FIG. 1. The trial was designed to include a Dose Escalation phase to identify a maximum tolerated dose (MTD) and then Expansion Cohorts treated at the MTD. As described in more detail below, the Dose Escalation phase included two dosing schedules. For Schedule A, IMGN632 was administered intravenously every three weeks (Q3W) on Day 1 of each 21-day cycle. For Schedule B, IMGN632 is administered intravenously two or three times every three weeks, i.e. on Days 1 and 8 of each 21-day cycle or on Days 1, 4, and 7 of each 21-day cycle.

### Subjects

Patients with recurrent or relapsed CD123+ AML or BPDCN per cohort are identified based on the following inclusion and exclusion criteria.
Inclusion Criteria:
   - Patients in dose escalation and all expansion cohorts except first relapse AML may have received up to three prior lines of therapy. Patients with relapsed AML (dose expansion only) received up to two prior lines of therapy.
   - Dose Escalation - Relapsed or refractory AML (excluding acute promyelocytic leukemia) or BPDCN, based on World Health Organization Classification. All patients enrolled on this study have CD123+ disease.
   - Dose Expansion Cohort #1 - Patients have relapse of CD123+ BPDCN. Patients with prior CD123-targeting agents are allowed as long as the blasts still have detectable CD123 expression.
   - Dose Expansion Cohort #2 - Patients have first relapse of CD123+ AML.
   - Dose Expansion Cohort #3 - Patients have relapse of CD123+ ALL.
   - Dose Expansion Cohort #4 - Patients have relapse of CD123+ "other" hematologic malignancies not included in the cohorts above (e.g., high-risk/very high-risk MDS, MPN, CMML, CML blast crisis). Other CD123+ malignancies may be considered.
Exclusion Criteria:
   - Patients who have available standard of care therapies are excluded.
   - AML patients with active central nervous system (CNS) disease are excluded.
   - Patients with a history of venous occlusive disease of the liver are excluded.
   - Patients with a history of Grade 3-4 capillary leak syndrome, or non-cardiac Grade edema were ineligible, e.g., related to SL-401 or other etiology are excluded.
   - Patients with a myocardial infarction within six months prior to enrollment or with New York Heart Association Class III or IV heart failure, uncontrolled angina, severe uncontrolled ventricular arrhythmias, or electrocardiographic evidence of acute ischemia or active conduction system abnormalities prior to study entry are excluded.
   - Patients who have received any anti-cancer therapy including chemotherapy, immunotherapy, radiotherapy, hormonal, biologic, or any investigational agents within 14 days or five half-lives, whichever is greater (with exception of hydroxyurea), prior to drug administration on this study are excluded.

### Treatment

Patients receive a premedication regimen prior to each IMGN632 infusion. The premedication includes (i) 25-50 mg diphenhydramine (IV or *per os* [PO]); (ii) 325-650 mg acetaminophen or paracetamol (IV or PO) and/or (iii) 8 mg dexamethasone (PO or IV). If individual patients required more intensive or alternative treatment to prevent infusion reactions (e.g., a different corticosteroid, different dose of any agent), the regimen may be modified according to standard institutional practice.

The planned treatment consists of two cycles (i.e., a total of six weeks), wherein patients' second doses are administered at least 21 days after their first doses. Additional cycles, for example up to 10 or more total, can be administered for patients deriving benefit from this regimen.

For purposes of this study, the period of safety observation extends from the time the patient give informed consent to participate in the study until the final safety follow-up visit. Patients who discontinue for reasons other than progressive disease (PD) undergo disease assessments (bone marrow aspirates or blood tests [complete blood count with differential]) every 12 weeks (± three weeks) until either documentation of PD, the initiation of a subsequent anti-cancer therapy, or for up to one year from the time of their last tumor assessment, whichever comes first. After documentation of PD or initiation of new anti-cancer therapy, the patient is contacted every 12 weeks (± three weeks) for the subsequent use of anti-cancer therapy as well as survival until one year from last patient's first dose of study drug (IMGN632).

### Pharmacokinetic assessments

Blood samples are collected at predetermined time points to assess the pharmacokinetics (PK) of IMGN632, total antibody, and free payload. Metabolites of IMGN632 are also evaluated.

### Safety assessments

Safety is assessed by reported/elicited adverse events (AEs), laboratory assessments including hematology and serum chemistry, vital signs, physical examination, and electrocardiogram/echocardiogram as indicated. The assessment of treatment-emergent AEs (TEAEs) included serious AEs (SAEs), AEs leading to study drug discontinuation, and AEs related to the study drug. All AEs occurring from informed consent until 30 days after last study drug administration were recorded regardless of the seriousness, severity, or relationship to study drug.

Patients who develop a dose-limiting toxicity (DLT) may continue treatment at a reduced dose level (a minimum reduction of at least one dose level) if the TEAE reverts to baseline or ≤ Grade 2. DLTs are defined in Table 4 below.

**Table 4: Dose-Limiting Toxicities (DLTs) and Adverse Events (AE)**

| TOXICITY | CRITERIA |
|---|---|
| Hematology | Failure of recovery to an absolute neutrophil count (ANC) of ≥ 0.5×10⁹/L and/or platelet count of ≥25×10⁹/L, when bone marrow otherwise indicates remission by 42 days after the first day of IMGN632. |
| | Aplasia (i.e., bone marrow cellularity <5%) that does not recover within 42 days after the first day of IMGN632. |
| | Lack of count recovery if active marrow is demonstrated is not considered a DLT. |
| Gastrointestinal | ≥ Common Terminology Criteria for Adverse Events (CTCAE) Grade 3 vomiting or nausea despite the use of optimal anti-emetic treatments. |
| | ≥ CTCAE Grade 3 diarrhea despite the use of optimal anti-diarrheal treatments |
| Renal | Serum creatine ≥ 3.0 x upper limit of normal [ULN] (except for isolated elevations - see below) |
| Hepatic | Bilirubine, alanine aminotransferase (ALT), or aspartate aminotransferase (AST) ≥ five x ULN (except for isolated elevations - see below) |
| | For any dose-limiting hepatic toxicity, evaluations should be performed to determine the underlying etiology and rule out drug-induced liver injury (Hy's Law). |
| Adverse Events that are NOT DLTs | Grade 3 fatigue, asthenia, anorexia, fever, or constipation |
| | Grade 3 nausea, vomiting, or diarrhea not requiring tube feeding, total parenteral nutrition (TPN), or hospitalization |
| | Grade 3 or 4 infection, bleeding, or other expected direct complications of cytopenias due to active leukemia |
| | Grade 3 or 4 febrile neutropenia |
| | Grade 3 infusion reaction including cytokine release syndrome (CRS), if successfully managed and which resolves within 72 hours |
| | Grade 3 or 4 tumor lysis syndrome (TLS) if it is successfully managed clinically and resolves within 7 days without end-organ damage |
| Other Adverse Events | Non-hematological toxicities of CTCAE ≥ Grade 3 are considered DLTs EXCEPT isolated Grade 3 elevations in biochemistry laboratory values without associated clinical symptoms that resolve to ≤ Grade 1 or baseline in ≤ 7 days. This includes electrolyte abnormalities that respond to medical intervention. |

### Anti-tumor activity

Response assessments are performed in bone marrow aspirates for differential and biomarker assessments taken on Cycle 1, Day 21 ± 7 days. Subsequent bone marrow aspirates are performed approximately every 1-2 cycles as clinically indicated, and at the 30-day follow-up visit. Data is collected in the event bone marrow aspirates are performed more frequently. No repeat bone marrow is necessary if lack of response (CR without minimal residual disease [CRMRD-], CR, CR with incomplete recovery [CRi], clinical CR [CRc; BPDCN only], or partial remission/response [PR]) or PD was unequivocally diagnosed from peripheral blood tests or if the bone marrow test is considered non-contributory by the Investigator at any time point.

### Example 2: Schedule A - IMGN632 administered once in a 21-day cycle

The starting dose for IMGN632 in Schedule A is 0.015 mg/kg. Doses from 0.015 mg/kg to 1.0 mg/kg were identified as outlined in Table 5.

**Table 5: Planned Schedule A Dose Escalation Cohorts**

| **Dose Escalation Cohorts** | **IMGN632 (mg/kg/dose)** | **Fold Dose Increase over Prior Dose Level** |
|---|---|---|
| 1 | 0.015 | -- |
| 2 | 0.045 | 3 |
| 3 | 0.09 | 2 |
| 4 | 0.18 | 2 |
| 5 | 0.3 | 1.67 |
| 6 | 0.45 | 1.5 |
| 7 | 0.67 | 1.5 |
| 8 | 1.0 | 1.5 |

### Results

Response criteria for AML and other Heme Malignancies except BPDCN:

For patients with AML and other heme malignancies except BPDCN, subjects were evaluated as having (i) complete remission (CR) without minimal residual disease (CRMRD-); (ii) complete remission, (iii) complete remission with incomplete recovery (CRi); (iv) morphologic leukemia-free state; (v) partial remission (PR); (vi) relapse following complete response; (vii) stable disease (SD); or (viii) progressive disease (PD). Patients are also evaluated as having CRh.

A complete remission (CR) for AML and other heme malignancies except BPDCN requires all of the following: morphologic CR < 5% blasts; absolute neutrophil count > 1,000/µL; platelets ≥ 100,000/µL; patient independent of transfusions; no residual evidence of active extramedullary disease; and MRD+ or unknown.

CR without minimal residual disease (CRMRD-) for AML and other heme malignancies except BPDCN includes all of the criteria for CR with negativity for a genetic marker by RT-qPCR, or CR with negativity by multi-parameter flow cytometry (MFC).

Complete remission with incomplete recovery (CRi) for AML and other heme malignancies except BPDCN meets requirements for CR except either ANC < 1,000/µL or platelets < 100,000/µL.

Morphologic leukemia-free state for AML and other heme malignancies except BPDCN includes bone marrow < 5% blasts in an aspirate with spicules; no blasts with Auer rods or persistence of extramedullary disease; and marrow should not merely be "aplastic"; at least 200 cells should be enumerated or cellularity should be at least 10%.

Partial remission (PR) for AML and other heme malignancies except BPDCN includes a decrease of at least 50% in the percentage of blasts to 5% to 25% in the bone marrow aspirate and the normalization of blood counts, as noted above.

Relapse following complete response for AML and other heme malignancies except BPDCN is defined as reappearance of leukemia blasts in the peripheral blood or the finding of more than 5% blasts in the bone marrow, not attributable to another cause (e.g., bone marrow regeneration after consolidation therapy (or extramedullary relapse).

Stable disease (SD) for AML and other heme malignancies except BPDCN is defined as the absence of CRMRD-, CR, CRi, PR, MLFS; and criteria for PD not met.

Progressive disease (PD) for AML and other heme malignancies except BPDCN includes evidence for an increase in bone marrow blast percentage and/or increase of absolute blast counts in the blood:
- Increased or persistent bone marrow disease without at least a 100% improvement (i.e., a doubling) in ANC to an absolute level (> 0.5 × 10⁹/L [500/µL], and/or platelet count to > 50 × 10⁹/L [50,000/µL] non-transfused):
   (A) > 50% increase in bone marrow blasts over baseline (a minimum 15 percentage point increase is required in cases with < 30% blasts at baseline); or
   (B) persistent bone marrow blast percentage of > 70% over at least 3 months
- > 50% increase in peripheral blasts (WBC × % blasts) to > 25 × 109/L (> 25 000/µL) (in the absence of differentiation syndrome); or
- New extramedullary disease.
   Response criteria for BPDCN:

For patients with BPDCN, subjects were evaluated as having CR; CRi; complete remission clinical (CRc); PR; SD; and PD. Patients are also evaluated as having CRh.

Complete remission (CR) includes normalization of peripheral blood and bone marrow; absence of active disease on positron emission tomography/computed tomography imaging; normal liver and spleen size without active nodules, and absence of skin involvement documented by examination and biopsy of previously affected areas.

CRi meets the requirements for CR except either ANC < 1,000/µL or platelets < 100,000/µL. CRc meets the requirements for CR except with residual microscopic skin disease.

PR includes greater than 50% decrease in bone marrow blasts (if blasts > 10% a study entry); greater than 50% decrease in the sum of the product of the diameters (SPDs) of up to six of the largest dominant nodal masses (if present at study entry); no increase in the size of other lymph nodes; greater than 50% decrease in SPD of spleen or liver nodules (if present at study entry); no increase in the size of the liver or spleen; and greater than 50% decrease in skin lesions (if present at study entry).

SD includes failure to achieve at least a PR in patients without bone marrow involvement and without evidence of disease progression in skin, lymph nodes, liver, or spleen. Finally, PD includes any new lymph nodes or new skin lesions; OR increase from nadir by > 50% of SPD of any single previously involved lymph node or total assessed lymph node masses; or OR > 50% increase from nadir in the SPD of liver or spleen nodules or > 50% increase in liver or spleen size.

### Schedule A Results

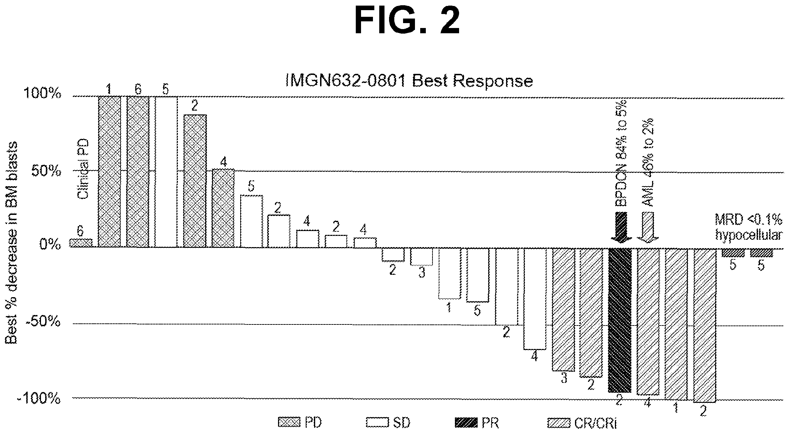

Patients in Cohorts 1-6 were treated with IMGN632 on Schedule A. As a measure of efficacy, decreases in bone marrow blasts were measured in each patient. As shown in **FIG. 2****,** five out of 25 evaluable patients had formal responses (CR or CRi), and two patients had non-formal responses (>30% reduction). In some patients, responses were observed after only 1 or 2 cycles. In some patients, responses improved (e.g., from a CRi to a CR) between 2 and 4 cycles of treatment.

Patient safety was evaluated in patients who received IMGN632 on Schedule A in Cohorts 1-6. Infusion-related reactions were identified in some patients. In particular, about 50% of patients showed Grade 1-2 infusion-related reactions, which was variable with steroid premedication. In some cases, patients developed SUSARs, including for some cases, e.g. tachycardia/hypertension, fever/headache. In some patients, a Grade 3 adverse effects, including febrile neutropenia, lung infection, and ALT/AST elevation were identified. Dose limiting toxicities were observed in Cohorts 5 and 6. In Cohort 5, myelosuppression and infection-related toxicities were also observed in 4 out of 5 patients, and 1 prolonged neutropenia (>42 days) was observed. Four deaths occurred shortly after the DLT period resulting from infection-related complications. In Cohort 6, liver toxicity (VOD) and neutropenia were observed. Two patients in Cohort 5 who had more than 5% blasts prior to treatment cleared their marrow (achieved MRD <0.1%), but both were hypocellular (<5%) and died prior to recovery. This provides evidence of IMGN632 activity, but in the context of excessive toxicity.

A summary of the results obtained using Schedule A in Cohorts 1-6 is provided in Table 6.

**Table 6: Schedule A results overview.**

| | Cohort 1 | Cohort 2 | Cohort 3 | Cohort 4 | Cohort 5 | Cohort 6 |
|---|---|---|---|---|---|---|
| Dose | 0.015 mg/kg (Q3W) | 0.045 mg/kg (Q3W) | 0.09 mg/kg (Q3W) | 0.18 mg/kg (Q3W) | 0.3 mg/kg (Q3W) | 0.45 mg/kg (Q3W) |

| # Patients | 3 | 12 | 8 | 7 | 5 | 2 |
|---|---|---|---|---|---|---|
| Results | CRi | CR, CRi, PR | CRi | CR | DLT: prolonged neutropenia; 4 infection-related deaths | DLT: veno-occlusive disease (VOD) |

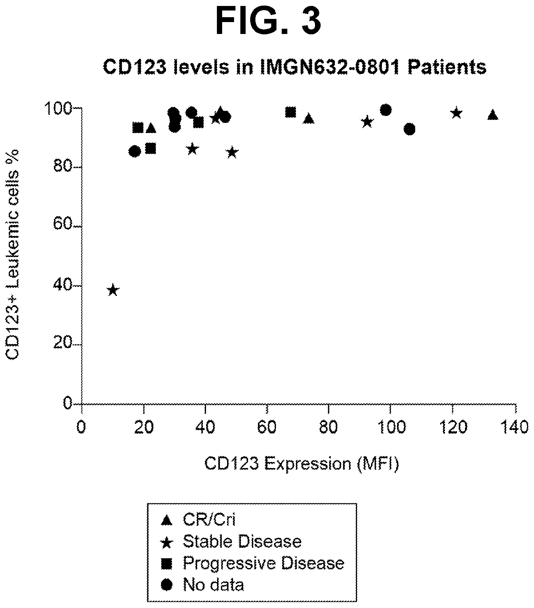

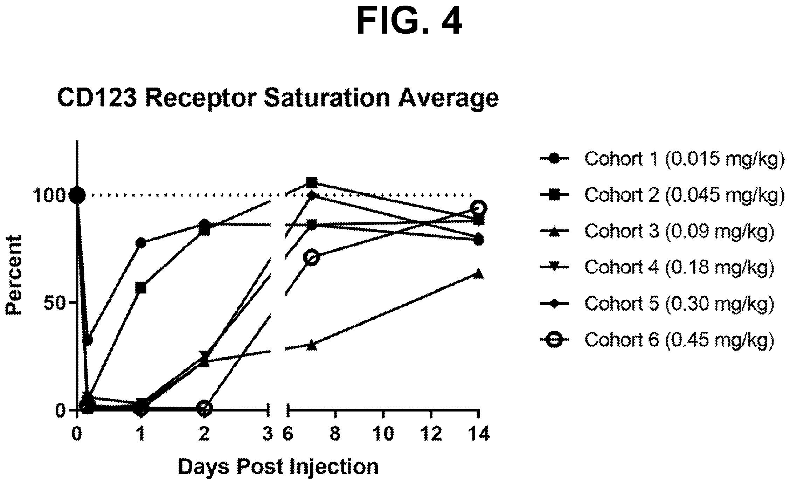

In additional studies, CD123 levels were measured. As shown in **FIG. 3****,** most patients had high CD123-uniformity (i.e., at least 80% of the leukemic cells in most patients were CD123+). In addition, the average CD123 receptor saturation was measured. As shown in **FIG. 4****,** complete saturation was observed with Cohorts 3 and above, but remains transient in most patients.

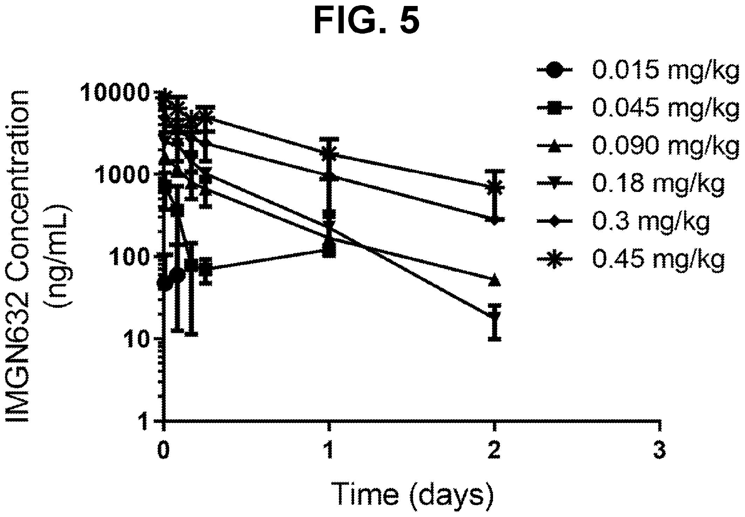

The pharmacokinetic (PK) parameters of IMGN632 were also measured. As shown in **FIG. 5****,** plasma antibody drug conjugate (ADC) measurements following a single intravenous infusion at doses ranging from 0.015 mg/kg through 0.45 mg/kg indicate that there was (i) sustained exposure through 48 hours post-infusion at doses ≥ 0.18 mg/kg; (ii) continued increase in maximal concentrations and exposure with increased dose; and (iii) consistent PK parameters within each dose cohort and following multiple dose cycles.

Based on these results, Expansion Cohorts were conducted with patients receiving doses of 0.045 mg/kg IMGN632 Q3W, 0.09 mg/kg IMGN632 Q3W, and 0.18 mg/kg IMGN632 Q3W. Patients with ANC <500/µL are treated with 0.09 mg/kg IMGN632 Q3W, and patients with ANC >500/µL are treated with 0.18 mg/kg IMGN632 Q3W.

### Example 3: Schedule B - IMGN632 administered multiple times in a 21-day cycle

In Schedule B, IMGN632 is administered in equal fractions across three day of a 21-day cycle (i.e., 1/3 of the total dose administered on each of Days 1, 4, and 8). The following doses are administered.

**Table 7: Planned Schedule B Dose Escalation Cohorts**

| **Dose Escalation Cohorts** | **IMGN632 Dose Per Administration** | **Days Administered** | **Total Dose in 21-Day Cycle** |
|---|---|---|---|
| B1 | 0.015 mg/kg | Days 1, 4, and 8 | 0.045mg/kg |
| B2 | 0.045 mg/kg | Days 1, 4, and 8 | 0.135 mg/kg |
| B3 | 0.09 mg/kg | Days 1, 4, and 8 | 0.27 mg/kg |

Dosing on Day 4 can be eliminated on Day 4 where the pK profile does not necessitate it. Thus, for example, 0.015 mg/kg can be administered on Days 1 and 8 for a total dose of 0.03 mg/kg in a 21-day cycle. In addition, 0.045 mg/kg can be administered on Days 1 and 8 for a total dose of 0.09 mg/kg in a 21-day cycle. In addition, 0.09 mg/kg can be administered on Days 1 and 8 for a total dose of 0.18 mg/kg in a 21-day cycle.

### Example 4: Therapeutic Efficacy of IMGN632

74 patients (67 AML, 7 BPDCN) received IMGN632 across nine dose-escalation cohorts on two schedules, with dosing escalated from 0.015-0.45 mg/kg on schedule A (n=61) and 0.015-0.06 mg/kg on days 1, 4, and 8 on schedule B (n=13). The median age of patients was 69 years (range 33-83). Forty-four percent had secondary AML, and 70% of classifiable AML patients were ELN adverse risk (32/46). Twenty-six percent were primary refractory to frontline therapy. Thirty-two percent were enrolled in first relapse, and forty-one percent had other relapsed-refractory disease. Sixty-eight percent of patients had received prior intense therapy, including stem cell transplant in 19%.

In the assessable AML population (n=66), 37 (55%) had a reduction in bone marrow blasts, and 13 (20%) achieved an objective response (3 CR, 8 CRi, 2 morphologic leukemia-free state (MLFS)) across a wide range of doses (0.045 to 0.3 mg/kg). Of note, the majority of responders (77%) had failed prior intensive therapies (including three with prior transplant), 62% had adverse ELN risk classification (including complex karyotype, ASXL1, RUNX1, and FLT3-ITD mutations), and 23% were primary refractory.

Of seven relapsed/refractory (R/R) BPDCN patients, three (43%) achieved an objective responses (CR, CRi, PR), two others had stable disease, and two had clinical progression. The patient with CR had previously had a partial response to SL-401, responded to CHOP, received a transplant and was refractory to decitabine with venetoclax; on IMGN632 this patient cleared bone marrow (28% to 0%) with one dose, and cleared skin (biopsy negative) and CT lesions with 2 doses. The patient with a CRi was refractory to SL-401, CLAG-M, and CLAG, and cleared bone marrow (37% to 0%), skin and CT lesions after one 0.045 mg/kg dose of IMGN632. The patient with a PR had previously had a partial response to SL-401; on IMGN632 this patient had complete clearance of bone marrow blasts (87% to 0%) and significant improvement in skin and CT lesions with one dose of IMGN632.

## Claims

1. An anti-CD123 immunoconjugate for use in a method for treating a hematologic malignancy in a human subject, the method comprising administering to the subject an anti-CD123 immunoconjugate comprising an anti-CD123 antibody or antigen-binding fragment thereof linked to an indolino-benzodiazepine (IGN) DNA-alkylator, wherein the immunoconjugate is administered at a dose of 0.045 mg/kg, wherein the anti-CD123 antibody or antigen-binding fragment in the immunoconjugate comprises:
a. a heavy chain variable region CDR1 comprising the amino acid sequence of SEQ ID NO: 5; a heavy chain variable region CDR2 comprising the amino acid sequence of SEQ ID NO: 6; and a heavy chain variable region CDR3 comprising the amino acid sequence of SEQ ID NO: 7; and
b. a light chain variable region CDR1 comprising the amino acid sequence of SEQ ID NO: 8; a light chain variable region CDR2 comprising the amino acid sequence of SEQ ID NO: 9; and a light chain variable region CDR3 comprising the amino acid sequence selected from the group consisting of: SEQ ID NO: 10,
wherein the immunoconjugate is for administration to the subject once per 21-day cycle.

2. The anti-CD123 immunoconjugate for use of claim 1, wherein the immunoconjugate is administered for at least 2 cycles, at least 3 cycles, at least 4 cycles, at least 5 cycles, at least 6 cycles, at least 7 cycles, at least 8 cycles, at least 9 cycles, or at least 10 cycles.

3. The anti-CD123 immunoconjugate for use of any one of claims 1-2, wherein the hematological malignancy is blastic plasmacytoid dendritic cell neoplasm (BPDCN), optionally wherein the BPDCN is front line BPDCN, relapsed BPDCN, and/or refractory BPDCN.

4. The anti-CD123 immunoconjugate for use of any one of claims 1-2, wherein the hematological malignancy is acute myeloid leukemia (AML); myelodysplastic syndrome (MDS), optionally, wherein the MDS is relapsed MDS and/or refractory MDS; acute lymphoblastic leukemia (ALL), optionally, wherein the ALL is B-cell ALL, relapsed ALL and/or refractory ALL; chronic myeloid leukemia in blast crisis/phase (BP-CML); chronic myelomonocytic leukemia (CMML), optionally, wherein the CMML is relapsed CMML and/or refractory CMML; or myelofibrosis (MF), optionally, wherein the MF is relapsed MF and/or refractory MF.

5. The anti-CD123 immunoconjugate for use of any one of claims 1-4, wherein the subject received at least one prior line of therapy.

6. The anti-CD123 immunoconjugate for use of any one of claims 1-5, wherein the subject has been pretreated with a corticosteroid prior to administration of the immunoconjugate, optionally wherein the corticosteroid is prednisone, prednisolone, methylprednisolone, beclomethasone, betamethasone, dexamethasone, fludrocortisone, hydrocortisone, or triamcinolone; or wherein the subject has been pretreated with diphenhydramine, acetaminophen, paracetamol, dexamethasone, or a combination thereof.

7. The anti-CD123 immunoconjugate for use of any one of claims 1-6, wherein the anti-CD123 antibody or antigen-binding fragment in the immunoconjugate comprises a VH comprising the amino acid sequence set forth in SEQ ID NO: 1, and a VL comprising the amino acid sequence set forth in SEQ ID NO: 2.

8. The anti-CD123 immunoconjugate for use of any one of claims 1-7, wherein the anti-CD123 antibody or antigen-binding fragment in the immunoconjugate comprises a heavy chain comprising the amino acid sequence set forth in SEQ ID NO: 3, and a light chain comprising the amino acid sequence set forth in SEQ ID NO: 4.

9. The anti-CD123 immunoconjugate for use of any one of claims 1-8, wherein the indolino-benzodiazepine (IGN) DNA-alkylator is DGN549-C.

10. The anti-CD123 immunoconjugate for use of any one of claims 1-9, wherein the immunoconjugate is in a pharmaceutical composition comprising immunoconjugates with the following structure: 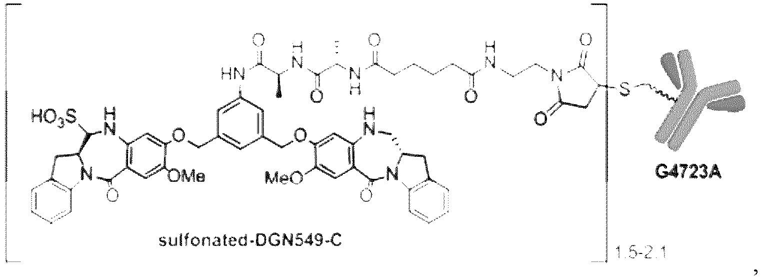 wherein G4723A comprises a heavy chain comprising the amino acid sequence set forth in SEQ ID NO: 3 and a light chain comprising the amino acid sequence set forth in SEQ ID NO: 4.

## Patentansprüche

1. Anti-CD123-Immunkonjugat zur Verwendung in einem Verfahren zur Behandlung einer hämatologischen Malignität in einem menschlichen Wesen, wobei das Verfahren die Verabreichung eines Anti-CD123-Immunkonjugats, das einen Anti-CD123-Antikörper oder ein Antigen-bindendes Fragment davon, an einen Indolino-Benzodiazepin (IGN)-DNA-Alkylator gebunden, aufweist, an das Wesen umfasst, wobei das Immunkonjugat in einer Dosis von 0,045 mg/kg verabreicht wird, wobei der Anti-CD123-Antikörper oder das Antigen-bindende Fragment in dem Immunkonjugat:
a. eine variable Region der schweren Kette CDR1, umfassend die Aminosäuresequenz von SEQ ID NO: 5; eine variable Region der schweren Kette CDR2, umfassend die Aminosäuresequenz von SEQ ID NO: 6; und eine variable Region der schweren Kette CDR3, umfassend die Aminosäuresequenz von SEQ ID NO: 7; und
b. eine variable Region der leichten Kette CDR1, umfassend die Aminosäuresequenz von SEQ ID NO: 8; eine variable Region der leichten Kette CDR2, umfassend die Aminosäuresequenz von SEQ ID NO: 9; und eine variable Region der leichten Kette CDR3, umfassend die Aminosäuresequenz, ausgewählt aus der Gruppe bestehend aus: SEQ ID NO: 10,
aufweist, wobei das Immunkonjugat zur Verabreichung an das Wesen einmal pro 21-Tage-Zyklus vorgesehen ist.

2. Anti-CD123-Immunkonjugat zur Verwendung nach Anspruch 1, wobei das Immunkonjugat für mindestens 2 Zyklen, mindestens 3 Zyklen, mindestens 4 Zyklen, mindestens 5 Zyklen, mindestens 6 Zyklen, mindestens 7 Zyklen, mindestens 8 Zyklen, mindestens 9 Zyklen oder mindestens 10 Zyklen verabreicht wird.

3. Anti-CD123-Immunkonjugat zur Verwendung nach einem der Ansprüche 1-2, wobei die hämatologische Malignität ein blastisches plasmazytoides dendritisches Zellneoplasma (BPDCN) ist, wobei das BPDCN optional ein BPDCN der ersten Linie, ein rezidiviertes BPDCN und/oder ein refraktäres BPDCN ist.

4. Anti-CD123-Immunkonjugat zur Verwendung nach einem der Ansprüche 1-2, wobei die hämatologische Malignität eine akute myeloische Leukämie (AML), ein myelodysplastisches Syndrom (MDS), gegebenenfalls wobei das MDS ein rezidiviertes MDS und/oder refraktäres MDS ist, eine akute lymphoblastische Leukämie (ALL), gegebenenfalls wobei die ALL eine B-Zell-ALL, rezidivierte ALL und/oder refraktäre ALL ist; chronische myeloische Leukämie in der Blasten-Krise/Phase (BP-CML); chronische myelomonozytäre Leukämie (CMML), gegebenenfalls, wobei die CMML rezidivierte CMML und/oder refraktäre CMML ist; oder Myelofibrose (MF), gegebenenfalls, wobei die MF rezidivierte MF und/oder refraktäre MF ist.

5. Anti-CD123-Immunkonjugat zur Verwendung nach einem der Ansprüche 1 bis 4, wobei das Wesen mindestens eine vorherige Therapielinie erhalten hat.

6. Anti-CD123-Immunkonjugat zur Verwendung nach einem der Ansprüche 1-5, wobei das Wesen vor der Verabreichung des Immunkonjugats mit einem Corticosteroid vorbehandelt wurde, wobei das Corticosteroid gegebenenfalls Prednison, Prednisolon, Methylprednisolon, Beclomethason, Betamethason, Dexamethason, Fludrocortison, Hydrocortison, oder Triamcinolon ist; oder wobei das Wesen mit Diphenhydramin, Acetaminophen, Paracetamol, Dexamethason, oder einer Kombination davon vorbehandelt wurde.

7. Anti-CD123-Immunkonjugat zur Verwendung nach einem der Ansprüche 1-6, wobei der Anti-CD123-Antikörper oder das Antigen-bindende Fragment in dem Immunkonjugat eine VH, die die in SEQ ID NO: 1 dargestellte Aminosäuresequenz aufweist, und eine VL, die die in SEQ ID NO: 2 dargestellte Aminosäuresequenz aufweist, aufweist.

8. Anti-CD123-Immunkonjugat zur Verwendung nach einem der Ansprüche 1-7, wobei der Anti-CD123-Antikörper oder das Antigen-bindende Fragment in dem Immunkonjugat eine schwere Kette, die die in SEQ ID NO: 3 dargestellte Aminosäuresequenz aufweist, und eine leichte Kette, die die in SEQ ID NO: 4 dargestellte Aminosäuresequenz aufweist, aufweist.

9. Anti-CD123-Immunkonjugat zur Verwendung nach einem der Ansprüche 1-8, wobei der Indolino-Benzodiazepin (IGN)-DNA-Alkylator DGN549-C ist.

10. Anti-CD123-Immunkonjugat zur Verwendung nach einem der Ansprüche 1-9, wobei das Immunkonjugat in einer pharmazeutischen Zusammensetzung vorliegt, die Immunkonjugate mit der folgenden Struktur umfasst: 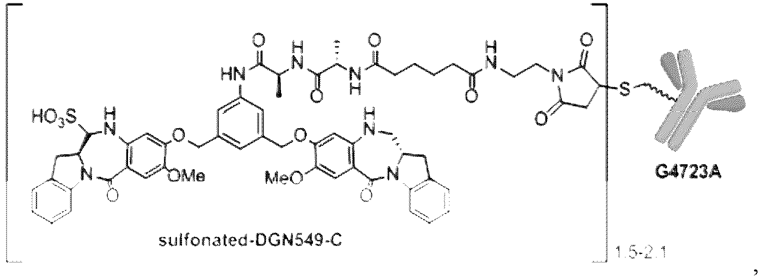 wobei G4723A eine schwere Kette, die die in SEQ ID NO: 3 dargestellte Aminosäuresequenz aufweist, und eine leichte Kette, die die in SEQ ID NO: 4 dargestellte Aminosäuresequenz aufweist, aufweist.

## Revendications

1. Immunoconjugué anti-CD123 utilisé dans une méthode de traitement d'une hémopathie maligne chez un sujet humain, la méthode consistant à administrer au sujet un immunoconjugué anti-CD123 comprenant un anticorps anti-CD123 ou un fragment se liant à l'antigène de celui-ci lié à un alkylateur d'ADN indolino-benzodiazépine (IGN), l'immunoconjugué étant administré à une dose de 0,045 mg/kg, dans lequel l'anticorps anti-CD123 ou le fragment se liant à l'antigène dans l'immunoconjugué comprend :
a. une région variable de chaîne lourde CDR1 comprenant la séquence d'acides aminés SEQ ID NO : 5 ; une région variable de chaîne lourde CDR2 comprenant la séquence d'acides aminés SEQ ID NO : 6 ; et une région variable de chaîne lourde CDR3 comprenant la séquence d'acides aminés SEQ ID NO : 7 ; et
b. une région variable de la chaîne légère CDR1 comprenant la séquence d'acides aminés de SEQ ID NO : 8 ; une région variable de la chaîne légère CDR2 comprenant la séquence d'acides aminés de SEQ ID NO : 9 ; et une région variable de la chaîne légère CDR3 comprenant la séquence d'acides aminés choisie dans le groupe constitué de : SEQ ID NO : 10,
dans lequel l'immunoconjugué est destiné à être administré au sujet une fois par cycle de 21 jours.

2. L'immunoconjugué anti-CD123 utilisé selon la revendication 1, dans lequel l'immunoconjugué est administré pendant au moins 2 cycles, au moins 3 cycles, au moins 4 cycles, au moins 5 cycles, au moins 6 cycles, au moins 7 cycles, au moins 8 cycles, au moins 9 cycles ou au moins 10 cycles.

3. L'immunoconjugué anti-CD123 utilisé dans l'une des revendications 1 et 2, dans lequel l'hémopathie maligne est un néoplasme blastique à cellules dendritiques plasmacytoïdes (BPDCN), éventuellement dans lequel le BPDCN est un BPDCN de première ligne, un BPDCN récidivant et/ou un BPDCN réfractaire.

4. L'immunoconjugué anti-CD123 utilisé dans l'une des revendications 1-2, dans lequel l'hémopathie maligne est une leucémie myéloïde aiguë (LMA), un syndrome myélodysplasique (SMD), optionnellement un SMD en rechute et/ou un SMD réfractaire, une leucémie lymphoblastique aiguë (LLA), optionnellement une LLA à cellules B, une LLA en rechute et/ou une LLA réfractaire, une leucémie myéloïde chronique en crise/phase blastique (LMCB), une leucémie myélomonocytaire chronique (LMC), optionnellement une LMCB en rechute et/ou une LMCB réfractaire ; leucémie myéloïde chronique en crise/phase blastique (LMC-PB) ; leucémie myélomonocytaire chronique (LMCM), optionnellement dans laquelle la LMCM est une LMCM en rechute et/ou une LMCM réfractaire ; ou myélofibrose (MF), optionnellement dans laquelle la MF est une MF en rechute et/ou une MF réfractaire.

5. L'immunoconjugué anti-CD123 utilisé dans l'une des revendications 1 à 4, dans lequel le sujet a reçu au moins une ligne de traitement antérieure.

6. L'immunoconjugué anti-CD123 utilisé dans l'une des revendications 1 à 5, dans lequel le sujet a été prétraité avec un corticostéroïde avant l'administration de l'immunoconjugué, éventuellement dans lequel le corticostéroïde est la prednisone, la prednisolone, la méthylprednisolone, la béclométhasone, la bétaméthasone, la dexaméthasone, la fludrocortisone, l'hydrocortisone, ou la triamcinolone ; ou dans lequel le sujet a été prétraité avec de la diphenhydramine, de l'acétaminophène, du paracétamol, de la dexaméthasone ou une combinaison de ces substances.

7. L'immunoconjugué anti-CD123 utilisé dans l'une des revendications 1 à 6, dans lequel l'anticorps anti-CD123 ou le fragment de liaison à l'antigène dans l'immunoconjugué comprend un VH comprenant la séquence d'acides aminés indiquée dans SEQ ID NO : 1, et un VL comprenant la séquence d'acides aminés indiquée dans SEQ ID NO : 2.

8. L'immunoconjugué anti-CD123 utilisé dans l'une des revendications 1 à 7, dans lequel l'anticorps anti-CD123 ou le fragment de liaison à l'antigène dans l'immunoconjugué comprend une chaîne lourde comprenant la séquence d'acides aminés décrite dans SEQ ID NO : 3, et une chaîne légère comprenant la séquence d'acides aminés décrite dans SEQ ID NO : 4.

9. L'immunoconjugué anti-CD123 utilisé dans l'une des revendications 1 à 8, dans lequel l'ADN-alkylateur indolino-benzodiazépine (IGN) est le DGN549-C.

10. L'immunoconjugué anti-CD123 utilisé dans l'une des revendications 1 à 9, dans lequel l'immunoconjugué est dans une composition pharmaceutique comprenant des immunoconjugués avec la structure suivante : 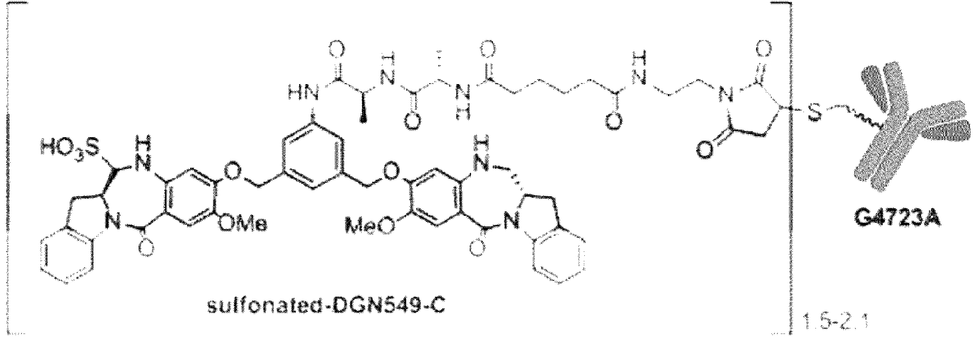 , dans lequel G4723A comprend une chaîne lourde comprenant la séquence d'acides aminés indiquée dans SEQ ID NO : 3 et une chaîne légère comprenant la séquence d'acides aminés indiquée dans SEQ ID NO : 4.
